# EUROPEAN PATENT SPECIFICATION

(11) **EP 4 403 177 B1**
(45) Date of publication and mention of the grant of the patent: **14.10.2026**
(21) Application number: 22869358.6
(22) Date of filing: 15.09.2022
(51) Int. Cl.: A61K 31/122, A61P 31/12, A61K 31/7056, A61P 31/14, A61K 31/426, A61K 45/06

(54) **NEW COMBINATION DRUG FOR TREATING CORONAVIRUS INFECTIONS, PHARMACEUTICAL COMPOSITION AND USE THEREOF**
NEUES KOMBINATIONSARZNEIMITTEL ZUR BEHANDLUNG VON CORONAVIRUSINFEKTIONEN, PHARMAZEUTISCHE ZUSAMMENSETZUNG UND VERWENDUNG DAVON
NOUVEAU MÉDICAMENT COMBINÉ POUR LE TRAITEMENT D'INFECTIONS À CORONAVIRUS, COMPOSITION PHARMACEUTIQUE ET UTILISATION ASSOCIÉE

(30) Priority: 15.09.2021 CN 202111082439; 03.12.2021 CN 202111466166
(43) Date of publication of application: 24.07.2024
(73) Proprietor: Oneglobe Holdings Limited, Hong Kong 999077 (HK)
(72) Inventor: LI, Chiang J, Norwood, MA 02062 (US)
(74) Representative: Haseltine Lake Kempner LLP
(86) International application number: PCT/CN2022/119159
(87) International publication number: WO 2023/040990

(56) References cited:
- WO-A1-2020/214716
- CN-A- 107 105 660
- GUEDES ISABELLA A., COSTA LEON S. C., DOS SANTOS KARINA B., KARL ANA L. M., ROCHA GREGÓRIO K., TEIXEIRA IURY M., GALHEIGO MARCELO : "Drug design and repurposing with DockThor-VS web server focusing on SARS-CoV-2 therapeutic targets and their non-synonym variants", SCIENTIFIC REPORTS, NATURE PUBLISHING GROUP, US, vol. 11, no. 1, 10 March 2021 (2021-03-10), US , pages 5543 - 20, XP093050035, ISSN: 2045-2322, DOI: 10.1038/s41598-021-84700-0
- AL-HORANI RAMI A., KAR SRABANI: "Potential Anti-SARS-CoV-2 Therapeutics That Target the Post-Entry Stages of the Viral Life Cycle: A Comprehensive Review", VIRUSES, vol. 12, no. 10, pages 1092, XP055798456, DOI: 10.3390/v12101092
- EUGENE MURATOV, ZAKHAROV ALEXEY: "Viribus Unitis: Drug Combinations as a Treatment Against COVID-19", CHEMRXIV, 20 April 2020 (2020-04-20), XP055727589, Retrieved from the Internet <URL:https://s3-eu-west-1.amazonaws.com/itempdf74155353254prod/12143355/Viribus_Unitis__Drug_Combinations_as_a_Treatment_Against_COVID-19_v1.pdf> [retrieved on 20200904], DOI: 10.26434/chemrxiv.12143355.v1

## Description

### FIELD OF THE INVENTION

The present invention relates to novel pharmaceutical compositions, combined drugs regimen and use thereof for treating or preventing coronavirus infection, especially SARS-CoV-2 infection and the acute respiratory infectious diseases or pneumonia (COVID-19) caused by SARS-CoV-2 infection.

### BACKGROUND OF THE INVENTION

COVID-19, which is caused by novel coronavirus (SARS-CoV-2) infection at the end of 2019 has been a global pandemic for more than 2 years, more than 6.5 million deaths worldwide as of September 2022. The characteristics of SARS-CoV-2, such as highly transmissible, have caused great difficulties in the treatment of infected people, and the prevention and control of the epidemic. It has also caused great losses to national economy, society and people's health of various countries. Vaccines and specific drug are the key to ending the global pandemic of the novel coronavirus. As the epidemic continues to spread, new mutant strains of SARS-CoV-2 that escape the mechanism of existing preventive vaccines and therapeutic medicine are constantly evolving. Many mutant strains have been appeared, including Alpha, Beta, Gamma, Delta, Epsilon, Zeta, Eta, Theta, Iota, Kappa, Lambda and Omicron. Among them, five mutant strains, namely Alpha, Beta, Gamma, Delta and Omicron, are listed as "Variant of Concern " (VOC) by WHO (https://www.who.int/en/activities/tracking-SARS-CoV-2-variants/). Wherein, Omicron mutant strain, including BA.1, BA.4, BA.5 and other descendant lineages, is highly infectious and is now the most major infectious strain. Vaccines as the main means for prevention and control of the epidemic at the present stage, the protection against SARS-CoV-2 mutant strains has been substantially reduced, and the level and duration of effective neutralizing antibodies produced have decreased. After the virus breaks through the immune protection by vaccines, patients may still progress to severe disease. Dealing with the rapidly mutating novel coronavirus is a common problem faced by global epidemic prevention and control. Effective and accessible means for fighting the epidemic at all stages of disease progression (including the treatment of patients in severe symptom, prevention the progression from mild symptom to severe symptom, prevention of onset after infection, and prevention of infection) is required. Therefore, safe and effective broad-spectrum anti-SARS-CoV-2 specific drugs are urgently needed.

At present, novel coronavirus therapeutic drugs mainly include three kinds, namely neutralizing antibodies, small molecule antiviral drugs and anti-cytokine storm.

Neutralizing antibodies: Neutralizing antibodies are a kind of drugs, that have received the most Emergency Use Authorization (EUA) by the US FDA, including GSK's Sotrovimab, Eli Lilly's Bamlanivimab/Etesevimab combination, Regeneron's Casirivimab/Imdevimab combination, and other drugs under research include AstraZeneca's AZD7442, Brii Biosciences' BRII-196/BRII-198, etc. Neutralizing antibodies can inhibit virus infection to normal cells by specifically binding to the virus spike protein, thereby achieving the purpose of prevention and treatment. The advantages of this kind of drugs lies in its high antigen specificity, that can accurately recognize the antigen of novel coronavirus, and low off-target effects. However, it is precisely because of its high specificity, the effectiveness of neutralizing antibodies for response to mutant strains has been substantially decreased. From market perspective, monoclonal antibodies are constrained by production capacity and production costs, and therefore monoclonal antibodies drugs are expensive. For example, the purchase price of Regeneron's neutralizing antibody by the US government is 2,100 dollars for a single dose. The pressure is very high for both government procurement and individual use. In addition, monoclonal antibody drugs need to be administered through intravenous infusion or subcutaneous injection, and under the supervision of professional medical workers, and therefore patients are required to go to the hospital for treatment, which is inconvenient and not suitable for the pandemic situation, such as for the promotion and use in high-risk groups. The three monoclonal antibody drugs that have been authorized by EUA are all aimed at mild symptoms to moderate symptoms patients, which means that the mild to moderate symptoms patients who do not need to be hospitalization are also required to go to the hospital for drug administration. And for the severe symptoms and critically ill patients who need to be hospitalized, since the clinical trials of neutralizing antibodies for severe symptoms and critically ill patients with the novel coronavirus have not yet shown therapeutic effects nor anti-inflammatory effect, monoclonal antibody drugs are not available to severe symptoms and critically ill patients either.

Small molecule antiviral drugs: mainly include nucleosides and 3CL protease inhibitors. The former mainly includes Gilead's Remdesivir, Merck's Molnupiravir, Genuine Biotech's Azvudine, Junshi Bio's VV116, etc.; The latter mainly includes Pfizer's Paxlovid (Consists of PF-07321332 and Ritonavir), Shionogi's S-217622, Shanghai Institute of Material Medica Chinese Academy of Sciences' DC402234 and SIM0417. The Nucleoside drugs have disadvantages such as low oral bioavailability, high toxicity and so on. For example, remdesivir is administered by intravenous infusion because of its low bioavailability and difficulty in achieving effective concentrations. The genotoxicity and mutagenic risks of Merck's anti-coronavirus drugs are still controversial, and the long-term safety is unknown. The long-term safety data of Pfizer's oral anti-SARS-CoV-2 drugs targeting 3CL protease is unknown, and the safety cannot be guaranteed. Small molecule antiviral drugs only target the virus itself and have no anti-inflammatory effect, so the applicable population is limited to patients with mild to moderate symptoms. Same as the neutralizing antibodies, the effects of small molecule antiviral drugs targeting SARS-CoV-2 polymerase and protease on the mutated strains are unclear, and from the perspective of virology, continuously mutating strains generally escape from such drugs targeting viral enzymes.

The third kind of drugs, anti-cytokine storm: these drugs have received less attention compared with neutralizing antibodies and small-molecule antiviral drugs, mainly because its mechanism of action is to target a specific inflammatory factor, and do not directly inhibit the virus, nor can it target all or most of the inflammatory factors. The two drugs that have been approved by FDA and EUA are the JAK inhibitor Baricitinib and the IL-6 inhibitor Tocilizumab, which have previously been approved for indications such as rheumatoid arthritis, and are only suitable for severe symptoms and critically ill hospitalized patients with COVID-19 and the therapeutic effect is very limited.

Among the drugs mentioned above, the second kind of drugs - small molecule antiviral drugs have multiple advantages, and also have some problems such as safety and anti-inflammatory properties. Therefore, in order to overcome the safety and anti-inflammatory problems of existing small molecule antiviral drugs, further exploration on such specific drugs research is urgently needed.

Nitazoxanide (NTZ) is a safe and orally bioavailable thiazolide, approved in the United States of America for treatment of *Cryptosporidium parvum* and *Giardia lamblia* infections, which is indicated for the treatment of transmissible gastroenteritis. This drug has been used in many countries, such as those in Africa and South America, and the results show that it is effective against parasitic infection with good safety. Tizoxanide (TIZ) is the main active metabolite of nitazoxanide *in vivo.* Nitazoxanide and tizoxanide were used as drugs to treat protozoal infections, and in later studies they have been found to inhibit the activity of cellular inflammation-related proteins including COX-2 (cyclooxygenase), NF-κB, p65, IκBα, ERK1/2 or p38MAPK, and therefore used to treat inflammatory-related diseases, including lipopolysaccharide (LPS) and other bacterial endotoxin-induced inflammatory diseases. Another study revealed that nitazoxanide has an inhibitory effect on the activity of acetyl-CoA carboxylase in HepG2 cells, and can have the effect of reducing obesity, lowering blood lipid level, preventing fatty liver and preventing atherosclerosis (CN110478357A). In addition, it is proved that nitazoxanide and tizoxanide can reduce the duration of symptoms associated with human respiratory syncytial virus, and other cell experimental results show that nitazoxanide or tizoxanide has antiviral activity of rotavirus, HBV, HCV, Dengue virus, yellow fever virus, Japanese encephalitis virus and HIV *in vitro* (Jean-François Rossignol, Romark Laboratories, L.C., 2014*;* CN108289961A); however, there is no data to support its antiviral activity *in vivo.* Nitazoxanide-based mechanism against viral infections is not yet clear. To date, nitazoxanide is not approved for the treatment of any viral infections in human.

Atovaquone (ATQ) is an orally bioavailable quinone compound approved in the United States, Europe, Japan and other countries for the treatment of parasitic pneumonia. It is effective against plasmodium, pneumocystis carinii, Babesia and toxoplasma gondii *in vivo.* So far, atovaquone is not approved for the treatment of any viral infections in humans.

Ribavirin is an antiviral, nucleoside analogue agent, approved by the U.S. Food and Drug Administration (FDA) for the treatment of hepatitis C, viral hemorrhagic fever, and severe lower respiratory tract infection caused by respiratory syncytial virus (RSV) (aerosol inhalation only). Although it has been shown to have inhibitory effects on a variety of other DNA and RNA viruses (such as herpes simplex virus, influenza virus, etc.) *in vitro,* its indications are generally limited due to lack of definitive clinical evidence. FDA clearly indicated in the approved drug label that ribavirin is not suitable for the treatment of influenza et al. (*COPEGUS*^{®} *(ribavirin) Tablets, Initial U.S. Approval: 2002*)*.* Ribavirin, alone or in combination with systemic glucocorticoids, has been tried for empiric treatment of severe acute respiratory syndrome (SARS) caused by SARS-CoV infection, but its clinical benefit has been disappointing (*WHO. Management of severe acute respiratory syndrome (SARS) 2003 Apr 11*). Nitazoxanide (NTZ), atovaquone (ATQ) and ribavirin (RBV) alone or in dual therapy NTZ+RBV are known for the treatment of coronavirus infections (see Al-Horani Rami A. et al. "Potential Anti-SARS-CoV-2 Therapeutics That Target the Post-Entry Stages of the Viral Life Cycle: A Comprehensive Review", Viruses, vol. 12, no. 10 , page 1092, XP055798456,DOI: 10.3390/v12101092).

### SUMMARY OF THE INVENTION

The present invention provides a novel pharmaceutical composition and a novel combined drug regimen for treating or preventing coronavirus infection, relates to a pharmaceutical composition or combinatorial drugs comprising the anti-protozoan infection drug nitazoxanide or tizoxanide, the nucleoside analogue ribavirin and/or the anti-parasitic drug atovaquone, as well as the use of such pharmaceutical composition or combinatorial drugs for inhibiting coronavirus infections, alleviating or reversing pneumonia, or preventing coronavirus infections. The references to the methods of treatment by therapy or surgery or in vivo diagnosis methods of this description are to be interpreted as references to compounds, pharmaceutical compositions and medicaments of the present invention for use in those methods.

In the first aspect, the present invention relates to a novel combinatorial drug and its use in the treatment or prevention of coronavirus infections, or its associated diseases or conditions, especially SARS-CoV-2 and its mutant strains infection, and the pneumonia that it causes.

In one embodiment, the combinatorial drug comprises: at least one drug selected from the group consisting of nitazoxanide, tizoxanide, and a pharmaceutically acceptable salt thereof; and at least one drug selected from the group consisting of ribavirin, a prodrug, metabolite, derivative, enantiomer, diastereomer, tautomer, or racemate thereof, and any pharmaceutically acceptable salt or ester of the foregoing, and/or at least one drug selected from the group consisting of atovaquone, a prodrug, metabolite, derivative, enantiomer, diastereomer, tautomer, or racemate thereof, or any pharmaceutically acceptable salt of the aforementioned.

In one embodiment, the combinatorial drug comprises at least one drug selected from nitazoxanide or tizoxanide or a pharmaceutically acceptable salt thereof, at least one drug selected from atovaquone or a pharmaceutically acceptable salt thereof, and at least one drug selected from ribavirin or a pharmaceutically acceptable salt or ester thereof.

In one embodiment, the combinatorial drug further comprises at least one other therapeutic agent or a combination thereof, and the other therapeutic agent or a combination thereof is other antiviral drugs, or other symptomatic treatments drugs for symptoms caused by coronavirus infection. In one embodiment, the combinatorial drug further comprises at least one other therapeutic agent or a combination thereof, non-limiting examples of the other therapeutic agent are selected from antiviral antibody drugs and other antiviral drugs, such as Favipiravir, Arbidol, Darunavir, or pharmaceutically acceptable salt or ester thereof.

In one embodiment, the combinatorial drug disclosed in present invention is used for the preparation of pharmaceutical composition for the treatment or prevention of coronavirus infection or its associated diseases or conditions, wherein the coronavirus is SARS-CoV-2 or its mutant strains, SARS, MERS, 229E, NL63, OC43 and/or HKU1, especially SARS-CoV-2 or its mutant strains. In some embodiments, the coronavirus is SARS-CoV-2 or its mutant strains thereof. In some embodiments, mutant strains of SARS-CoV-2 include, but are not limited to Alpha(B.1.1.7), Beta(B.1.351), Gamma(P.1), Delta(B.1.617.2), Epsilon(B.1.427/B.1.429), Zeta(P2), Eta(B.1.525), Theta(P.3), Iota(B.1.526), Kappa(B.1.617.1), Lambda(C.37), Mu(B.1.621) and Omicron (B.1.1.529 and its descendants lineages BA.1, BA.1.1, BA.2, BA.2.12.1, BA.2.75, BA.3, BA.4, BA.4.6, BA.5, XE). In some embodiments, the associated disease or condition is pneumonia.

In one embodiment, the combinatorial drug disclosed herein is used to treat, alleviate or relieve at least one symptom of above-described diseases or conditions associated thereof, non-limiting examples of such symptoms including those selected from cough, decreased appetite, asthma, dyspnea, fever, general malaise, dizziness, nausea, diminished or lost sense of smell, and pneumonia.

Described herein is a method for the treatment or prevention of coronavirus (in particular SARS-CoV-2 and mutant strains thereof) infections or associated diseases or conditions thereof, wherein the method comprises administering a therapeutically effective amount of the novel combinatorial drug of the first aspect mentioned above to a subject in need. In some embodiments, the associated diseases or conditions are acute respiratory infection disease or pneumonia. Wherein the respective active ingredients of the combinatorial drug can be administered simultaneously or sequentially to a subject in need thereof.

The method at least comprises administering to a subject in need thereof: a therapeutically effective amount of at least one drug selected from nitazoxanide or tizoxanide or a pharmaceutically acceptable salt thereof, a therapeutically effective amount of at least one atovaquone or a pharmaceutically acceptable salt thereof, and a therapeutically effective amount of at least one ribavirin or a pharmaceutically acceptable salt or ester thereof. In some embodiments, nitazoxanide or tizoxanide or a pharmaceutically acceptable salt thereof, atovaquone or a pharmaceutically acceptable salt thereof and ribavirin or a pharmaceutically acceptable salt or ester thereof can be administered simultaneously or sequentially to a subject in need thereof.

In one embodiment, the subjects in need are common, severe, or critically ill patients caused by coronavirus infection. In one embodiment, the subjects in need are mild to moderate symptoms patients or severe symptoms patients caused by coronavirus infection.

The method further comprises administering a therapeutically effective amount of at least one other therapeutic agent to a subject in need, wherein the other antiviral agents are other antiviral drugs or other symptomatic treatments drugs of symptoms caused by coronavirus infection.

In one embodiment, the coronaviruses are SARS-CoV-2 or its mutants, SARS, MERS, 229E, NL63, OC43 and/or HKU1, especially SARS-CoV-2 or its mutants. In some embodiments, the mutant strains of SARS-CoV-2 include, but are not limited to, Alpha (B.1.1.7), Beta (B.1.351), Gamma (P.1), Delta (B.1.617.2), Epsilon (B.1.427/B.1.429), Zeta (P.2), Eta (B.1.525), Theta (P3), Iota (B.1.526), Kappa (B.1.617.1), Lambda (C.37), Mu (B.1.621) and Omicron (B.1.1.529 and its descendants lineages BA.1, BA.1.1, BA.2, BA.2.12.1, BA.2.75, BA.3, BA.4, BA.4.6, BA.5, XE).

The method for preventing diseases or conditions caused by or associated with coronavirus infection refers to chemoprevention/drug prevention.

In some embodiments, the administration of the combinatorial drugs disclosed by the present invention can be used to alleviate at least one symptom of above-described diseases or conditions, wherein the non-limiting examples of the at least one symptom selected from the group consisting of cough, decreased appetite, asthma, dyspnea, fever, general malaise, dizziness, nausea, diminished or lost sense of smell, and pneumonia.

In some embodiments, wherein nitazoxanide is administered in an oral formulation.

In some embodiments, wherein atovaquone is administered in an oral formulation.

In some embodiments, the ribavirin can be administered in an oral formulation, an inhalation formulation or an injection formulation. In some embodiments, ribavirin is administered in an aerosol inhalation formulation directly via the respiratory tract. In some embodiments, ribavirin is administered in an oral formulation.

Also described are methods for treating or preventing coronavirus (especially SARS-CoV-2 and mutant strains thereof) infection or its associated diseases or conditions, the methods comprise administering nitazoxanide, atovaquone, and ribavirin to a subject in need thereof, wherein the nitazoxanide is administered to a subject in need thereof at a total daily dose of about 100 mg to about 3000 mg, the atovaquone is administered to a subject in need thereof at a total daily dose of about 20 mg to about 3000 mg, and the ribavirin is administered to a subject in need thereof at a total daily dose of about 20 mg to about 2500 mg, wherein nitazoxanide, atovaquone and ribavirin can be administered once daily (QD), twice daily (BID), three times daily (TID) or four times daily (QID), respectively.

In some embodiments, nitazoxanide is administered to a subject at a total daily dose of about 100 mg to about 2500 mg, or about 300 mg to about 1800 mg. In some embodiments, nitazoxanide is administered once daily (QD), twice daily (BID), three times daily (TID), or four times daily (QID).

In some embodiments, nitazoxanide is administered to a subject in a single dose of about 100 mg to about 1000 mg. In some embodiments, nitazoxanide is administered to a subject once daily (QD), twice daily (BID), three times daily (TID), or four times daily (QID).

In some embodiments, nitazoxanide is administered to a subject in a single dose from about 100 mg to about 550 mg, once daily (QD), twice daily (BID), three times daily (TID) or four times daily (QID).

In some embodiments, nitazoxanide is administered to a subject in a dose of about 100 mg to about 600mg BID, about 100 mg to about 600mgTID, and about 100 mg to about 500mg QID.

In some embodiments, atovaquone is administered to a subject at a total daily dose of about 20 mg to about 2500 mg, or about 300 mg to about 1800 mg. In some embodiments, atovaquone can be administered once daily (QD), twice daily (BID), three times daily (TID), or four times daily (QID).

In some embodiments, atovaquone is administered to a subject in a single dose of about 60 mg to about 1000 mg. In some embodiments, atovaquone is administered once daily (QD), twice daily (BID), three times daily (TID), or four times daily (QID).

In some embodiments, atovaquone is administered to a subject in a single dose from about 100 mg to about 540 mg, twice daily (BID), three times daily (TID) or four times daily (QID).

In some embodiments, atovaquone is administered to a subject in dose of about 60 mg to about 600mg BID, about 60 mg to about 600mgTID, and about 100 mg to about 500mg QID.

In some embodiments, ribavirin is administered to a subject at a total daily dose of about 20 mg to about 1500 mg. In some embodiments, ribavirin can be administered once daily (QD), twice daily (BID), three times daily (TID), or four times daily (QID).

In some embodiments, ribavirin is administered to a subject in a single dose of about 20 mg to about 500 mg. In some embodiments, ribavirin is administered once daily (QD), twice daily (BID), three times daily (TID), or four times daily (QID).

In some embodiments, ribavirin is administered to a subject in a single dose from about 20 mg to about 400 mg, once daily (QD), twice daily (BID), three times daily (TID) or four times daily (QID).

In some embodiments, ribavirin is administered to a subject in a dose of about 20 mg to about 500mg BID, about 20 mg to about 400mg TID, and about 20 mg to about 400mg QID.

In some embodiments, nitazoxanide, atovaquone and ribavirin can be administered simultaneously for the first dose. In some embodiments, the interval period between the first administration of sequentially administered nitazoxanide, atovaquone and ribavirin ranges from about 0 hours to about 16 hours. In some embodiments, the interval period between the first administration of sequentially administered nitazoxanide, atovaquone and ribavirin is at least 0 hour, at least 0.1 hour, at least 0.2 hour, at least 0.3 hour, at least 0.4 hour, at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 13 hours, at least 14 hours, at least 15 hours and/or at least 16 hours.

In some embodiments, nitazoxanide, atovaquone and ribavirin are administered to a subject twice daily (BID), respectively. The first dose of Nitazoxanide, atovaquone and ribavirin can be administered simultaneously, or the first dose of nitazoxanide, atovaquone and ribavirin can be administered sequentially with interval period to each other within the range of about 0 hours to about 8 hours, wherein nitazoxanide is administered in a single dose of about 100 mg to about 550 mg, atovaquone is administered in a single dose of about 100 mg to about 550 mg, and ribavirin is administered in a single dose of about 30 mg to about 400 mg.

In some embodiments, nitazoxanide, atovaquone and ribavirin are administered to a subject three times daily (TID), respectively. The first dose of Nitazoxanide, atovaquone and ribavirin can be administered simultaneously, or the first dose of nitazoxanide, atovaquone and ribavirin can be administered sequentially with interval period to each other within the range of about 0 hours to about 4 hours, wherein nitazoxanide is administered in a single dose of about 100 mg to about 550 mg, atovaquone is administered in a single dose of about 100 mg to about 550 mg, and ribavirin is administered in a single dose of about 30 mg to about 200 mg.

In some embodiments, nitazoxanide, atovaquone and ribavirin are administered to a subject four times daily (QID), respectively. The first dose of Nitazoxanide, atovaquone and ribavirin can be administered simultaneously, or the first dose of nitazoxanide, atovaquone and ribavirin can be administered sequentially with interval period to each other within the range of about 0 hours to about 3 hours, where nitazoxanide is administered in a single dose of about 100 mg to about 550 mg, atovaquone is administered in a single dose of about 100 mg to about 550 mg, and ribavirin is administered in a single dose of about 30 mg to about 200 mg.

In the third aspect, the present invention relates to a novel pharmaceutical composition and its use for the treatment or prevention of coronavirus infections, or its associated diseases or conditions, especially SARS-CoV-2 infection and its mutant strains infection and the pneumonia that it causes. The pharmaceutical composition comprises active pharmaceutical ingredient a) a therapeutically effective amount of nitazoxanide or tizoxanide or a pharmaceutically acceptable salt thereof, b) a therapeutically effective amount of atovaquone or a pharmaceutically acceptable salt thereof and c) a therapeutically effective amount of ribavirin or a pharmaceutically acceptable salt or ester thereof.

In some embodiments, the pharmaceutical composition comprises the active pharmaceutical ingredient and at least one pharmaceutically acceptable excipient, carrier or diluent; The active pharmaceutical ingredient comprises: a) at least one selected from nitazoxanide or tizoxanide or a pharmaceutically acceptable salt thereof, b) at least one atovaquone or a pharmaceutically acceptable salt thereof, and c) at least one ribavirin or a pharmaceutically acceptable salt or ester thereof. In some embodiments, wherein the active pharmaceutical ingredient comprises nitazoxanide, atovaquone and ribavirin. In some embodiments, wherein the weight ratio of nitazoxanide: atovaquone: ribavirin is about (0.1-10): (0.1-10): 1. In some embodiments, wherein the weight ratio of nitazoxanide: atovaquone: ribavirin is about 1.5:1.5:1, about 2:2:1, about 2.5:2.5:1, about 3:3:1, about 10:10:3, about 3.5:3.5:1, about 4:4:1, about 4.5:4.5:1 or about 5:5:1 and any range value between any two aforementioned ratios. In some embodiments, the pharmaceutical composition contains about 50 mg to about 1200 mg of nitazoxanide per unit formulation, about 50 mg to about 1200 mg of atovaquone per unit formulation, and ribavirin about 1 mg to about 500 mg per unit formulation. In some embodiments, wherein the unit formulation of the pharmaceutical composition contains about 60 mg to about 600 mg of nitazoxanide, about 60 mg to about 600 mg of atovaquone, and about 10 mg to about 200 mg of ribavirin. In some embodiments, wherein the unit formulation of the pharmaceutical composition contains about 111 mg of nitazoxanide, about 111 mg of atovaquone, and about 33 mg of ribavirin. In some embodiments, the weight ratio of the active pharmaceutical ingredient and the pharmaceutically acceptable excipient, carrier or diluent is about (0.1-4):1. In some embodiments, the weight content of the pharmaceutically acceptable excipient, carrier or diluent of the pharmaceutical composition is about 0.1-90% (w/w).

In some embodiments, the pharmaceutical composition is tablet, capsule or suspension.

Also disclosed herein is a method for treating or preventing coronavirus infection or diseases or conditions associated with it, wherein the method comprises administering the novel pharmaceutical composition in the third aspect mentioned above to a subject in need thereof. In some embodiments, the associated disease or condition is pneumonia.

In some embodiments, the pharmaceutical composition is administered orally.

The method for treating or preventing coronavirus infection or associated diseases or conditions thereof comprises administering the pharmaceutical composition 1-4 times a day to a subject in need thereof with 1-5 unit(s) formulation each time. In some embodiments, the method comprises administering the pharmaceutical composition 2-4 times a day to a subject in need thereof with 2-4 unit(s) formulation each time.

In one embodiment, the subjects in need thereof are common, severe, or critically ill patients caused by coronavirus infection. In one embodiment, the subjects in need are mild to moderate symptoms patients or severe symptoms patients caused by coronavirus infection.

The method further comprises administering a therapeutically effective amount of at least one other therapeutic agent to a subject in need, wherein the other therapeutic agent is another antiviral agent or other symptomatic treatment drugs of symptoms caused by coronavirus infection. In one embodiment, the non-limiting examples of the other therapeutic agents are selected from antiviral antibody drugs, other antiviral drugs such as Favipiravir, Arbidol, Darunavir or a pharmaceutically acceptable salt or ester thereof.

In one embodiment, the coronavirus is SARS-CoV-2 or its mutant strains, SARS, MERS, 229E, NL63, OC43 and/or HKU1, especially SARS-CoV-2 or its mutant strains. In some embodiments, the mutant strains of SARS-CoV-2 include, but are not limited to Alpha (B.1.1.7), Beta (B.1.351), Gamma (P.1), Delta (B.1.617.2), Epsilon (B.1.427/B.1.429), Zeta (P.2), Eta (B.1.525), Theta (P.3), Iota (B.1.526), Kappa (B.1.617.1), Lambda (C.37) and Omicron (B.1.1.529 and its descendants lineages BA.1, BA.1.1, BA.2, BA.2.12.1, BA.2.75, BA.3, BA.4, BA.4.6, BA.5, XE).

The method for preventing diseases or conditions caused by or associated with coronavirus infection refers to chemoprevention/drug prevention.

In some embodiments, the administration of the combinatorial drugs disclosed by the present invention can be used to alleviate at least one symptom of above-described diseases or conditions associated thereof, where the non-limiting examples of the at least one symptom selected from the group consisting of cough, decreased appetite, asthma, dyspnea, fever, general malaise, dizziness, nausea, diminished or lost sense of smell, and pneumonia.

In the fifth aspect, the present invention relates to a novel combinatorial drug and its use in the treatment or prevention of coronavirus infections, or its associated diseases or conditions, especially SARS-CoV-2 and its mutant strains infection and the pneumonia that it causes, wherein the combinatorial drug comprises at least one drug selected from nitazoxanide or tizoxanide or a pharmaceutically acceptable salt thereof, and at least one drug selected from atovaquone or a pharmaceutically acceptable salt thereof.

In one embodiment, the combinatorial drug may further comprise at least one selected from nucleoside analogues or a pharmaceutically acceptable salt or ester thereof. In one feature, the nucleoside analogue is a compound selected from the group consisting of Sofosbuvir, Ribavirin, Remdesivir or a combination thereof.

In one embodiment, the combinatorial drug comprises at least one other therapeutic agent or a combination thereof, and the other therapeutic agent or a combination thereof is other antiviral drugs, or other symptomatic treatments for symptoms caused by coronavirus infection. In one embodiment, the combinatorial drug further comprises at least one other therapeutic agent or a combination thereof, the non-limiting examples of the other therapeutic agent are selected from antiviral antibody drugs, and other antiviral drugs such as Favipiravir, Arbidol, Darunavir or a pharmaceutically acceptable salt or ester thereof.

In one embodiment, the combinatorial drug disclosed in the present invention is used to treat, alleviate or relieve at least one symptom of the associated diseases or conditions, non-limiting examples of such symptoms including those selected from cough, decreased appetite, asthma, dyspnea, fever, general malaise, dizziness, nausea, diminished or lost sense of smell, and pneumonia.

In one embodiment, t the combinatorial drug disclosed in present invention is used for the preparation of pharmaceutical composition for treating or preventing coronavirus infections or its associated diseases or conditions, wherein the coronavirus is SARS-CoV-2 or its mutant strains, SARS, MERS, 229E, NL63, OC43 and/or HKU1, especially SARS-CoV-2 or its mutant strains. In some embodiments, the associated disease or condition is pneumonia.

Also disclosed is a method for the treatment or prevention of coronavirus (in particular SARS-CoV-2 and mutant strains thereof) infections or its associated diseases or conditions, where the method comprises administering a therapeutically effective amount of the novel combinatorial drug of the fifth aspect mentioned above to a subject in need. The associated diseases or conditions are acute respiratory infection disease or pneumonia. Wherein the respective active ingredients of the combinatorial drug can be administered simultaneously or sequentially to a subject in need thereof.

The method at least comprises administering to a subject in need thereof a therapeutically effective amount of at least one drug selected from nitazoxanide or tizoxanide or a pharmaceutically acceptable salt thereof, and a therapeutically effective amount of at least one atovaquone or a pharmaceutically acceptable salt thereof. In some embodiments, nitazoxanide or tizoxanide or a pharmaceutically acceptable salt thereof, and atovaquone or a pharmaceutically acceptable salt thereof can be administered simultaneously or sequentially to a subject in need thereof.

In one embodiment, the subject in need thereof are common, severe, or critically ill patients caused by coronavirus infection.

Herein disclosed are methods for the treatment or prevention of coronavirus (especially SARS-CoV-2) infections or its associated diseases or conditions, the methods comprise administering nitazoxanide and atovaquone to a subject in need thereof, wherein nitazoxanide is administered to a subject in need thereof at a total daily dose ranging from about 100 mg to about 3000 mg and atovaquone is administered to a subject in need thereof at a total daily dose ranging from about 20 mg to about 3000 mg, wherein nitazoxanide and atovaquone can be administered once daily (QD), twice daily (BID), three times daily (TID) or four times daily (QID), respectively.

In some embodiments, nitazoxanide is administered to a subject at a total daily dose of about 100 mg to about 2500 mg, or about 300 mg to about 1800 mg. In some embodiments, nitazoxanide can be administered once daily (QD), twice daily (BID), three times daily (TID), or four times daily (QID).

In some embodiments, nitazoxanide is administered to a subject in a single dose of about 100 mg to about 1000 mg. In some embodiments, nitazoxanide is administered once daily (QD), twice daily (BID), three times daily (TID), or four times daily (QID).

In some embodiments, nitazoxanide is administered to a subject in a single dose from about 100 mg to about 550 mg, twice daily (BID), three times daily (TID) or four times daily (QID).

In some embodiments, nitazoxanide is administered to a subject in dose of about 100 mg to about 600 mg BID, about 100 mg to about 600 mg TID, and about 100 mg to about 500 mg QID.

In some embodiments, nitazoxanide is administered to a subject in a dose of about 100 mg to about 500 mg TID or about 100 mg to about 500 mg QID.

In some embodiments, atovaquone is administered to a subject at a total daily dose of about 20 mg to about 2500 mg, or about 300 mg to about 1800 mg. In some embodiments, atovaquone can be administered to a subject once daily (QD), twice daily (BID), three times daily (TID), or four times daily (QID).

In some embodiments, atovaquone is administered to a subject in a single dose of about 60 mg to about 1000 mg. In some embodiments, atovaquone is administered once daily (QD), twice daily (BID), three times daily (TID), or four times daily (QID).

In some embodiments, atovaquone is administered to a subject in a single dose from about 100 mg to about 540 mg, twice daily (BID), three times daily (TID) or four times daily (QID).

In some embodiments, atovaquone is administered to a subject in a dose of about 60 mg to about 600mg BID, about 60 mg to about 600 mg TID, and about 100 mg to about 500mg QID.

In some embodiments, atovaquone is administered to a subject in a dose of about 100 mg to about 540 mg TID or 100 mg to about 540 mg QID.

In some embodiments, nitazoxanide and atovaquone can be administered simultaneously for the first dose. In some embodiments, the interval period between the first administration of sequentially administered nitazoxanide and atovaquone ranges from about 0 hours to about 16 hours. In some embodiments, the interval period between the first administration of sequentially administered nitazoxanide and atovaquone is at least 0 hour, at least 0.1 hour, at least 0.2 hour, at least 0.3 hour, at least 0.4 hour, at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 13 hours, at least 14 hours, at least 15 hours and/or at least 16 hours.

The method further comprises administering a therapeutically effective amount of at least one other therapeutic agent to a subject in need thereof, wherein the other therapeutic agent is another antiviral agent or other symptomatic treatment drug of symptoms caused by coronavirus infection.

In one embodiment, the coronavirus is SARS-CoV-2 or its mutant strains, SARS, MERS, 229E, NL63, OC43 and/or HKU1, especially SARS-CoV-2 or its mutant strains, and the associated disease or condition is pneumonia. In some embodiments, the mutant strains of SARS-CoV-2 include, but are not limited to Alpha (B.1.1.7), Beta (B.1.351), Gamma (P.1), Delta (B.1.617.2), Epsilon (B.1.427/B.1.429), Zeta (P.2), Eta (B.1.525), Theta (P.3), Iota (B.1.526), Kappa (B.1.617.1), Lambda (C.37), Mu(B.1.621) and Omicron (B.1.1.529 and its descendants lineages BA.1, BA.1.1, BA2, BA2.12.1, BA.2.75, BA.3, BA.4, BA.4.6, BA.5, XE).

The methods for preventing diseases or conditions caused by or associated with coronavirus infection refers to chemoprevention/drug prevention.

In some embodiments, the administration of the combinatorial drugs disclosed by the present invention can be used to alleviate at least one symptom of above-described diseases or conditions associated thereof, wherein the non-limiting examples of the at least one symptom are selected from the group consisting of cough, decreased appetite, asthma, dyspnea, fever, malaise, dizziness, nausea, diminished or lost sense of smell, and pneumonia.

In the seventh aspect, the present invention relates to a novel pharmaceutical composition and its use in the treatment or prevention of coronavirus infections, or its associated diseases or conditions, especially SARS-CoV-2 and its mutant strains infection and the pneumonia that it causes, wherein the pharmaceutical composition comprises the active pharmaceutical ingredient and at least one pharmaceutically acceptable excipient, carrier or diluent. The active pharmaceutical ingredient comprise a) a therapeutically effective amount of nitazoxanide or a pharmaceutically acceptable salt thereof, b) a therapeutically effective amount of atovaquone or a pharmaceutically acceptable salt thereof. In some embodiments, the mutant strains of SARS-CoV-2 include, but are not limited to Alpha (B.1.1.7), Beta (B.1.351), Gamma (P.1), Delta (B.1.617.2), Epsilon (B.1.427/B.1.429), Zeta (P.2), Eta (B.1.525), Theta (P.3), Iota (B.1.526), Kappa (B.1.617.1), Lambda (C.37), Mu(B.1.621) and Omicron (B.1.1.529 and its descendants lineages BA.1, BA.1.1, BA.2, BA.2.12.1, BA.2.75, BA.3, BA.4, BA.4.6, BA.5, XE).

In one embodiment, the active pharmaceutical ingredient is composed of nitazoxanide and atovaquone in a fixed ratio. In one embodiment, the fixed ratio refers to the weight ratio of nitazoxanide and atovaquone that is about 1:0.1 to about 1:10. In one embodiment, the fixed ratio refers to the weight ratio of nitazoxanide and atovaquone that is about 1:0.1, about 1:0.2, about 1:0.5, about 1:0.8, about 1:1, about 1:1.2, about 1:1.5, about 1:2, about 1:5, about 1:10, or any range value between any two aforementioned ratios. In a preferred embodiment, the fixed ratio refers to the weight ratio of nitazoxanide and atovaquone that is about 1:1.

In one embodiment, the pharmaceutical composition is formulated as a tablet.

In one embodiment, the pharmaceutical composition is formulated as a capsule.

In one embodiment, the pharmaceutical composition is formulated as a suspension.

In one embodiment, the unit formulation of the pharmaceutical composition contains about 50 mg to about 1200 mg of nitazoxanide per unit formulation, and about 50 mg to about 1200 mg of atovaquone per unit formulation. In one embodiment, the unit formulation of the pharmaceutical composition contains about 60 mg to about 500 mg of nitazoxanide per unit formulation and about 100 mg to about 500 mg of atovaquone per unit formulation. In a preferred embodiment, nitazoxanide is 165 mg per unit formulation and atovaquone is 165 mg per unit formulation in the unit formulation of the pharmaceutical composition.

In one embodiment, the pharmaceutical composition is administered to a subject in need thereof in 1-5 unit(s) formulation each time, 1-4 times a day. In one specific embodiment, the pharmaceutical composition is administered to a subject in need thereof in 2-4 units formulation each time, 2-4 times a day.

Describe herein are method for the treatment or prevention of coronavirus (especially SARS-CoV-2 and its mutant strains) infections or its associated diseases or conditions, wherein the method comprises administering the novel pharmaceutical composition of the seventh aspect mentioned above to a subject in need. The associated diseases or conditions is acute respiratory infection disease or pneumonia.

The method comprises administering the pharmaceutical composition 1-4 times per day to a subject in need thereof in 1-5 unit(s) formulation each time, wherein in the unit formulation of the pharmaceutical composition, the fixed weight ratio of nitazoxanide: atovaquone is about 1:0.1 to about 1:10, preferably about 1:1.

The method comprises administering the pharmaceutical composition 1-4 times per day to a subject in need thereof in 1-5 unit(s) formulation each time, wherein the unit formulation of the pharmaceutical composition contains about 50 mg to about 1200 mg of nitazoxanide, and about 50 mg to about 1200 mg of atovaquone.

The method comprises administering the pharmaceutical combination 3 times a day to a subject in need thereof in 2 units formulation each time, wherein the unit formulation of the pharmaceutical composition contains about 165 mg of nitazoxanide and about 165 mg of atovaquone.

In one embodiment, the subjects in need are common, severe, or critically ill patients caused by coronavirus infection.

The method further comprises simultaneously or sequentially administering another antiviral drug to a subject in need thereof, such as nucleoside analogues or a pharmaceutically acceptable salt or ester thereof. In one embodiment, the nucleoside analogue is a compound selected from the group consisting of Sofosbuvir, Ribavirin, Remdesivir or a combination thereof.

The method further comprises administering a therapeutic effective amount of at least one other therapeutic agent to a subject in need thereof, wherein the other therapeutic agents are other antiviral drugs, or other symptomatic treatment drugs for symptoms caused by coronavirus infection. In one embodiment, the non-limiting examples of the other therapeutic agents are selected from antiviral antibody drugs, and other antiviral drugs such as Favipiravir, Arbidol, Darunavir or a pharmaceutically acceptable salt or ester thereof.

In one embodiment, the coronavirus is SARS-CoV-2 or its mutant strains, SARS, MERS, 229E, NL63, OC43 and/or HKU1, especially SARS-CoV-2 or its mutant strains, and the associated diseases or conditions is pneumonia.

The methods for preventing diseases or conditions caused by or associated with coronavirus infection refers to chemoprevention/drug prevention.

In some embodiments, the administration of the pharmaceutical composition disclosed by the present invention can be used to alleviate at least one symptom of above-described diseases or conditions associated thereof, where the non-limiting examples of the at least one symptom are selected from the group consisting of cough, decreased appetite, asthma, dyspnea, fever and pneumonia.

The foregoing and other objects, aspects, features and advantages of the present invention are further apparent from the following description and the claims.

### BRIEF DESCRIPTION OF FIGURES

Figure 1 illustrates the inhibitory activity of the pharmaceutical composition of the present invention (**Anticov-1**) against the novel coronavirus.
Figure 2 illustrates the inhibition rates of six experimental drugs against SARS-CoV-2 in Vero E6 cells at different concentrations.
Figure 3 illustrates the Cytotoxic Effect of **Anticov-1** on Vero E6 cells at different concentrations. The observation results of **Anticov-1** incubated with Vero E6 cells for 24 hours are shown in Figure 3(a). The observation results of **Anticov-1** incubated with cells for 48 hours are shown in Figure 3(b).
Figure 4 is the plasma concentration of nitazoxanide in different test groups with time after a single administration. 1-NTZ, 4-NTZ and 5-NTZ respectively represent the plasma concentration of NTZ in group 1, group 4 and group 5.
Figure 5 is the plasma concentration of atovaquone in different test groups with time after a single administration. 2-ATQ, 4-ATQ and 5-ATQ respectively represent the plasma concentration of ATQ in group 2, group 4 and group 5.
Figure 6 is the plasma concentration of ribavirin in different test groups with time after a single administration. 3-RBV, 4-RBV and 5-RBV respectively represent the plasma concentration of RBV in group 3, group 4 and group 5.
Figure 7 is the schematic diagram of the role of the pharmaceutical composition of the present invention in inhibiting activation of STAT3, the master switch of inflammation storm, (one of the mechanisms of action of the pharmaceutical composition of the present invention to treat the novel coronavirus) in the pathological mechanism of the novel coronavirus.
Figure 8 is the test result of **Anticov-1** blocking IL-6-mediated activation of STAT3 in human blood cells.
Figure 9 is the test result of **Anticov-1** blocking IL-6-mediated activation of TNF-α in human blood cells.
Figure 10 illustrates the inhibitory rate of **Anticov-1** to 4 strains (WIV04, Omicron, Delta, Beta) of SARS-CoV-2 and the EC₅₀ of each strain in Vero E6 cells under different concentrations.

### DETAILED DESCRIPTION OF THE INVENTION

### I. I. definition

As used in the description and claims, the singular form "a", "an", or "the" includes plural references, unless the context clearly dictates otherwise. For example, the term "a cell" includes a plurality of cells including mixtures thereof.

As used herein, "about" is used herein to modify a numerical value above and below the stated value by a variance of 10%. For example, "about 1" means "0.9 to 1.1", "about 30" means "27 to 33", "about 2%" means "1.8% to 2.2%", "about 2% to 3%" means "1.8% to 3.3%", "about 3% to about 4%" means "2.7% to 4.4%", "about 1:1" means "1.1:1 to 1:1.1".

As used herein, "range value between two ratios" means that in two ratios, the same value part is taken as the base, and different value parts are respectively used as the upper and lower limits of the optional value range. For example, the range value between the two ratios of about 1.5:1.5:1 and about 2:2:1 means that take the same value "1" as the base, components with different values have optional value ranges, that is, different value parts as the upper limit and lower limit of the optional range value, and that is, mean any specific ratio included in the range of about (1.5~2):(1.5~2):1. For another example, the range value between about 1:0.8 and about 1:1.2 refers to any specific ratio included in the range of about 1:(0.8~1.2).

Unless otherwise defined, all technical and scientific terms used herein have the same meaning as generally understood by a person of ordinary skill in the art to which the present invention belongs.

As described herein, the "mild symptoms patient" or "mild patient", "moderate symptoms patient" or "common patient", "severe moderate patient" or " severe patient" are respectively corresponding to the judgment criteria adopted in clinical diagnosis and treatment practice, in which the two terms before and after connected with "or" have the same meaning and can be used interchangeably. "mild to moderate symptoms patients" as used herein refers to patients with mild symptoms to moderate symptoms. In some embodiments, the patient diagnostic criteria are as follows:
(-) Mild: Those with mild clinical symptoms and no imaging manifestation of pneumonia.
( 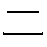 ) Common: Those with fever, respiratory tract symptoms, etc., and imaging manifestations of pneumonia.
( 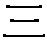 ) Severe: Adults that meet any of the following:
   1. Shortness of breath, RR≥30 times/minute;
   2. Under resting state, the oxygen saturation when inhaling air≤93%;
   3. Arterial partial pressure of oxygen (PaO₂)/inhaled oxygen concentration (FiO₂)≤300 mmHg (1 mmHg = 0.133kPa); In high-altitude (more than 1000 meters above sea level) areas, PaO₂/FiO₂ should be corrected according to the following formula: PaO₂/FiO₂×[760/atmospheric pressure (mmHg)];
   4. Progressive aggravation of clinical symptoms, and pulmonary imaging shows that the lesion has significantly progressed >50% within 24 to 48 hours.

The term "effective amount" of an active agent refers to the amount that is sufficient to elicit the required biological response. As understood by those of ordinary skill in the art, the effective amount of a compound of the present invention is vary depending on such factors as the desired biological endpoints, the pharmacokinetics of the compound, the disease being treated, the mode of administration, and the patient.

The term "carrier" which is administered with a compound refers to a non-limiting diluent, solubilizer, binder, disintegrant, lubricant, adjuvant, excipients or vehicle, etc...

The term "solubilizer" when used herein refers to any pharmaceutically acceptable surfactant. Non-limiting examples of solubilizers include, but are not limited to, sodium lauryl sulfate (SLS) or sodium dodecyl sulfate (SDS), polyoxyethylene polyoxypropylene copolymers (e.g. poloxamer 188, Pluronic F68), polyoxyethylene sorbitan fatty acid esters (polysorbate, preferably polyoxyethylene sorbitan monooleate (Tween 80^{™})) or polyoxyethylene sorbitan monolaurate (Tween 20^{™}), certain lipids, such as lecithin (eg, dimyristoylphosphatidylcholine (DMPC)).

The term "diluent" refers to a chemical compound used to dilute a compound of interest prior to delivery. Diluents can also be used to stabilize compounds. Non-limiting examples of diluents include, but are not limited to, starch, carbohydrate, disaccharide, sucrose, lactose, lactose monohydrate, polysaccharide, compressible sugar, cellulose, cellulose ether, hydroxypropyl cellulose, sugar alcohol, xylitol, sorbitol, mannitol, maltitol, microcrystalline cellulose, calcium carbonate or sodium carbonate, calcium hydrogen phosphate, dibasic calcium phosphate dehydrator and tricalcium phosphate.

The term " binder" refers to any pharmaceutically acceptable film when used herein, which can be used to bind together the active and inert components of the carrier together to maintain cohesive and discrete portions. Non-limiting examples of binders include, but are not limited to, hydroxypropylcellulose, hydroxypropylmethylcellulose, povidone, copovidone, and ethylcellulose.

The term "disintegrant" refers to a substance that, when added to a solid preparation, facilitates its break-up or disintegration after administration and allow the active ingredient to be released as effectively as possible so as to dissolve rapidly. Non-limiting examples of disintegrants include, but are not limited to, corn starch, sodium carboxymethyl starch, croscarmellose sodium, crospovidone, microcrystalline cellulose, modified corn starch, povidone, pregelatinized starch and alginic acid.

The term "lubricant" refers to an excipient added to a powder mixture to prevent the compressed powder substance from sticking to the device during tableting or packaging process. It facilitates ejection of the tablet from the mold and improves the flowability of the powder. Non-limiting examples of lubricants include, but are not limited to, magnesium stearate, stearic acid, silicon dioxide, fats, calcium stearate, polyethylene glycol, sodium stearyl fumarate or talc; and "solubilizer" include, but are not limited to, fatty acids, lauric acid, oleic acid and C8/C10 fatty acids.

The term "glidant" refers to substances used in tablets or capsules formulation to improve fluidity and produce an anti-caking effect. Non-limiting examples of glidants include, but are not limited to, colloidal silicon dioxide, talc, atomized silica, starch, starch derivatives and expansive soils.

The term "film coating" refers to a thin, uniform film on the surface of a substrate (eg tablet). Film coatings are especially useful for protecting active ingredients from photodegradation. Non-limiting examples of film coatings include, but are not limited to, polyvinyl alcohol based, hydroxyethyl cellulose, hydroxypropyl methyl cellulose, sodium carboxymethyl cellulose, polyethylene glycol 4000 and phthalic acid cellulose film coating.

The terms "treatment" or "treating" (of) a disease or disorder refer to a method of reducing, delaying or improving such a condition before or after it occurs. Treatment may be directed at one or more effects or symptoms of the disease and/or the underlying pathology. The treatment can be any reduction and can be, but is not limited to, the complete eliminate of the disease or symptoms of the conditions. As compared with an equivalent untreated control, such degree of reduction or prevention as measured by any standard technique is at least 5%, 10%, 20%, 30%, 40%, 50%, 60%, 70%, 80%, 90%, 95%, or 100%.

The term "therapeutic effective amount" means what is commonly accepted in this art. The term generally refers to the amount of a compound or composition that will elicit a necessary biological or medical response in a cell, tissue, system, animal or human body. For example, if a given clinical treatment is considered effective when the measurable parameters associated with the disease or disorder are reduced by at least approximately 25%, the therapeutic effective amount of the drug used to treat the disease or disorder is the amount necessary to reduce the parameter by at least approximately 25%.

As used herein, the term "subject" refers to any animal (e.g., a mammal), including, but not limited to humans, non-human primates, rodents, and the like, which is to be the recipient of a particular treatment or prevention scheme. Typically, the terms "subject" "patient" and "individual(s) in need of prophylactic protection from infection or population(s) at high risk" are used interchangeably herein and refer to human subject(s).

"Composition (Combine)" or "combination (combinatorial)" therapy or treatment as used herein refers to the administration of at least two different drugs for the treatment of a disorder, condition or symptom, for example, viral pneumonia, viral infection, coronaviral pneumonia and the like. Such combination therapy comprises administering one drug before, at the time of, and/or after the administration of the other drug. The interval period between administrations of the different drugs is up to several weeks, but is more commonly within 48 hours, and most commonly within 24 hours.

The compounds of the present invention may form salts which are also within the scope of this invention. Reference to a compound of the present invention herein is understood to include reference to salts thereof, unless otherwise indicated. The term "salt(s)", as employed herein, denotes acidic and/or basic salts formed with inorganic and/or organic acids and bases. In addition, when the compound of the present invention contains both a basic moiety (such as, but not limited to, a pyridine or imidazole) and an acidic moiety (such as, but not limited to, a carboxylic acid), zwitterions ("inner salts") may be formed and are included within the term "salt(s)" as used herein. Pharmaceutically acceptable (i.e., non-toxic, physiologically acceptable) salts are preferred, although other salts are also useful, e.g., in isolation or purification steps which may be employed during preparation.

"Chemoprevention" as used herein refers to the administration of the combinatorial drug regimen provided herein to asymptomatic persons with a viral infection, or to persons whose viral infection test is negative but are considered to have a possibility of viral infection by epidemiologic history analysis, or persons whose viral infection test is negative but are considered to have a viral infection like pneumonia by other medical diagnosis (such as medical imaging analysis, etc.), as prophylactic measures, to prevent or mitigate the pathogenicity of the viral infection. The term "EC₅₀" as used herein refers to concentration for 50% of maximal effect, which refers to the concentration that can cause 50% of the maximum effect. The term "CC₅₀" as used herein refers to the drug concentration at which 50% of the cells become diseased.

### II. Combinatorial drugs

The present invention provides a novel combinatorial drug and its use in the treatment or prevention of coronavirus (such as SARS-CoV-2) infection or associated diseases or conditions thereof (such as pneumonia, COVID-19). Also disclosed are methods for treating or preventing a coronavirus infection (such as SARS-CoV-2 and its mutants), or associated diseases or conditions thereof (such as pneumonia, COVID-19), comprising administering to a subject in need thereof a therapeutically effective amount of the combinatorial drug described herein.

The novel combinatorial drug provided by the present invention, comprises: at least one thiazolide compound selected from the group consisting of nitazoxanide (NTZ), tizoxanide or derivatives thereof, and any pharmaceutically acceptable salt or ester of the foregoing or any solvate of the foregoing; at least one quinone compound selected from atovaquone (ATQ) or its derivatives, any pharmaceutically acceptable salt or ester of the foregoing or any solvate of the foregoing; and/or at least one nucleoside analogue selected from ribavirin (RBV) or derivatives thereof, any pharmaceutically acceptable salt or ester of the foregoing or any solvate of the foregoing.

In one embodiment, the novel combinatorial drug comprises at least one thiazolide compound selected from the group consisting of nitazoxanide (NTZ), tizoxanide or derivatives thereof, and any pharmaceutically acceptable salt or ester of the foregoing or any solvate of the foregoing; at least one quinone compound selected from atovaquone (ATQ) or its derivatives, any pharmaceutically acceptable salt or ester of the foregoing or any solvate of the foregoing; and at least one nucleoside analogue selected from ribavirin (RBV) or its prodrugs, metabolites, derivatives, enantiomers, diastereomers, tautomers, racemates, or any pharmaceutically acceptable salt or ester of the foregoing.

In one embodiment, the novel combinatorial drug comprises at least one thiazolide compound selected from the group consisting of nitazoxanide (NTZ), tizoxanide or derivatives thereof, and any pharmaceutically acceptable salt or ester of the foregoing or any solvate of the foregoing, and at least one quinone compound selected from atovaquone (ATQ) or its derivatives, any pharmaceutically acceptable salt or ester of the foregoing or any solvate of the foregoing.

In one embodiment, the combinatorial drug further comprises at least one other therapeutic agent or a combination thereof, and the other therapeutic agent or a combination thereof is other antiviral drugs, or other symptomatic treatments for symptoms caused by coronavirus infection. In one embodiment, the non-limiting examples of other therapeutic agents are selected from antiviral antibody drugs, and other antiviral drugs such as Favipiravir, Arbidol, Darunavir or a pharmaceutically acceptable salt or ester thereof.

In one embodiment, the present invention provides the use of the combinatorial drug in the preparation of pharmaceutical compositions for the treatment or prevention of coronavirus infection or associated diseases or conditions thereof, where the coronavirus includes, but is not limited to, SARS-CoV-2 or its mutant strains, SARS, MERS, 229E, NL63, OC43 and HKU1, especially SARS-CoV-2 or its mutant strains. In some embodiments, Alpha(B.1.1.7), Beta(B.1.351), Gamma(P.1), Delta(B.1.617.2), Epsilon(B.1.4278.1.429), Zeta(P.2), Eta(B.1.525), Theta(P.3), Iota(B.1.526), Kappa(B.1.617.1), Lambda(C.37), Mu(B.1.621) and Omicron (B.1.1.529 and its descendants lineages BA.1, BA.1.1, BA.2, BA.2.12.1, BA.2.75, BA.3, BA.4, BA.4.6, BA.5, XE). In some embodiments, the associated disease or condition is pneumonia.

### III. Pharmaceutical Compositions

The present invention provides a novel pharmaceutical Compositions and its use in the treatment or prevention of coronavirus (such as SARS-CoV-2) infection or associated diseases or conditions thereof (such as pneumonia, COVID-19). Also disclosed are methods for treating or preventing a coronavirus infection (such as SARS-CoV-2 and its mutants), or associated diseases or conditions thereof (such as pneumonia, COVID-19), comprising administering to a subject in need of a therapeutically effective amount of the pharmaceutical compositions described herein.

In one aspect, the novel pharmaceutical composition of the invention comprises the active pharmaceutical ingredient and at least one pharmaceutically acceptable excipient, carrier or diluent; The active pharmaceutical ingredient comprises: a) at least one selected from nitazoxanide or a pharmaceutically acceptable salt thereof, b) at least one atovaquone or a pharmaceutically acceptable salt thereof, and c) at least one ribavirin or a pharmaceutically acceptable salt or ester thereof.

In some embodiments, wherein the active pharmaceutical ingredient comprises nitazoxanide, atovaquone and ribavirin. In some embodiments, wherein the weight ratio of nitazoxanide: atovaquone: ribavirin is about (0.1-10):(0.1-10):1. Optionally, wherein the weight ratio of nitazoxanide: atovaquone: ribavirin is about 1.5:1.5:1, about 2:2:1, about 2.5:2.5:1, about 3:3:1, about 10:10:3, about 3.5:3.5:1, about 4:4:1, about 4.5:4.5:1, about 5:5:1 and any range value between any aforementioned two ratios.

In one embodiment, the weight ratio of the active pharmaceutical ingredient to the pharmaceutically acceptable excipient, carrier or diluent is about (0.1-4):1.

In one embodiment, the weight content of the pharmaceutically acceptable excipient, carrier or diluent in the pharmaceutical composition is about 0.1-90% (w/w).

In some embodiments, the pharmaceutical composition is formulated as a tablet, capsule or suspension. In some embodiments, pharmaceutical composition formulated as a tablet.

In one embodiment, the unit formulation of the pharmaceutical composition contains about 50 mg to about 1200 mg of nitazoxanide per unit formulation, about 50 mg to about 1200 mg of atovaquone per unit formulation, and ribavirin about 1 mg to about 500 mg per unit formulation. Optionally, wherein the unit formulation of the pharmaceutical composition contains about 60 mg to about 600 mg of nitazoxanide per unit formulation, about 60 mg to about 600 mg of atovaquone per unit formulation, and about 10 mg to about 200 mg of ribavirin per unit formulation. Optionally, wherein the unit formulation of the pharmaceutical composition contains about 111 mg nitazoxanide per unit formulation, about 111 mg atovaquone per unit formulation, and about 33 mg ribavirin per unit formulation.

In one embodiment, the pharmaceutical composition can be administered to a subject in need thereof with 1, 2, 3, 4 or 5 unit(s) each time, once a day (QD), twice a day (BID), three times a day (TID) or four times a day (QID).

In another aspect, the novel pharmaceutical composition of the invention comprises active pharmaceutical ingredients a) a therapeutically effective amount of nitazoxanide or a pharmaceutically acceptable salt thereof, b) a therapeutically effective amount of atovaquone or a pharmaceutically acceptable salt thereof, and at least one pharmaceutically acceptable excipient, carrier or diluent.

In one embodiment, the active pharmaceutical ingredient is composed of nitazoxanide and atovaquone in a fixed ratio. The fixed ratio refers to the weight ratio of nitazoxanide: atovaquone is about 1:0.1 to about 1:10. Preferably, the fixed ratio refers to the weight ratio of nitazoxanide: atovaquone is about 1:0.1, about 1:0.2, about 1:0.5, about 1:0.8, about 1:1, about 1:1.2, about 1:1.5, about 1:2, about 1:5, about 1:10, or any range value between any aforementioned two ratios. Particularly preferred, the fixed ratio refers to the weight ratio of nitazoxanide: atovaquone is about 1:1.

The pharmaceutical composition is formulated as tablet, capsule or suspension.

In one embodiment, the unit formulation of pharmaceutical composition contains about 50 mg to about 1200 mg of nitazoxanide per unit formulation, and about 50 mg to about 1200 mg of atovaquone per unit formulation. Preferably, wherein the unit formulation of the pharmaceutical composition contains about 60 mg to about 500 mg of nitazoxanide per unit formulation, and about 60 mg to about 500 mg of atovaquone per unit formulation. Preferably, wherein the unit formulation of the pharmaceutical composition contains about 80 mg to about 250 mg of nitazoxanide per unit formulation, and about 80 mg to about 250 mg per of atovaquone unit formulation. Preferably, wherein the unit formulation of the pharmaceutical composition contains about 100 mg to about 200 mg nitazoxanide per unit formulation, and about 100 mg to about 200 mg atovaquone per unit formulation. Preferably, wherein the unit formulation of the pharmaceutical composition contains about 111 mg nitazoxanide per unit formulation, about 111 mg atovaquone per unit formulation. Particularly preferred, wherein the unit formulation of the pharmaceutical composition contains about 165 mg nitazoxanide per unit formulation, about 165 mg atovaquone per unit formulation.

In one embodiment, the pharmaceutical composition can be administered to a subject in need thereof with 1, 2, 3, 4 or 5 unit(s) each time, once a day (QD), twice a day (BID), three times a day (TID) or four times a day (QID).

### Excipient

The pharmaceutical composition provided by the invention is administered by oral. The present invention provides a pharmaceutical composition comprising a solid dispersion, wherein the solid dispersion contains nitazoxanide, atovaquone, ribavirin as described herein and one or more pharmaceutically acceptable excipients or carriers. In some embodiments, the pharmaceutically acceptable excipients or carriers include, but are not limited to, diluents, solubilizers, disintegrants, lubricants, binders, glidants, and combinations thereof. This composition can be prepared by the method well known in the pharmaceutical field.

The pharmaceutical composition can be administered orally in single or multiple doses. Administration can be done by tablet, capsule, suspension, etc. In one embodiment, the pharmaceutical composition of the present invention is a compound preparation, and the compound preparation is in the form of the tablet. In another embodiment, the tablet is a compressed tablet. When preparing a pharmaceutical composition comprising a solid as described herein, the active ingredient is usually diluted with an excipient and/or encapsulated in a carrier, and the carrier can be in the form of a capsule, tablet, sachet, paper or other container. When the excipient acts as a diluent, it can be a solid, semi-solid or liquid material (as above), which acts as a vehicle, carrier or medium for the active ingredient.

In one embodiment, the pharmaceutical composition comprises solubilizer selected from the group consisting of sodium lauryl sulfate (SLS, sodium dodecyl sulfate (SDS)), poloxamer 188 (Pluronic F68), polyoxyethylene sorbitan monooleate (Tween 80^{™})) or polyoxyethylene sorbitan monolaurate (Tween 20^{™}), 1,2-dimyristoyl-sn-glycero-3-phosphocholine (DMPC), and their combinations. In some embodiments, the weight content of the solubilizer in the pharmaceutical composition is about 0.1-3%w/w.

In one of the embodiments, the pharmaceutical composition contains SLS, wherein the amount of SLS is from about 0.1% to about 3% w/w, or from about 0.1% to about 2.5% w/w, or from about 0.5% to about 3% w/w, or from about 0.5% to about 2.5% w/w, or from about 0.5% to about 2% w/w, or from about 1% to about 3% w/w, or from about 1% to about 2% w/w. In particular embodiment, the amount of SLS is about 0.1%, or about 0.5%, or about 1%, or about 2%, or about 2.5%, or about 3% w/w.

In another embodiment, the pharmaceutical composition contains poloxamer 188, wherein the amount of poloxamer 188 is from about 0.1% to about 3% w/w, or from about 0.1% to about 2.5% w/w, or from about 0.5% to about 3% w/w, or from about 0.5% to about 2.5% w/w, or from about 0.5% to about 2% w/w, or from about 1% to about 3% w/w, or from about 1% to about 2% w/w. In particular embodiment, the amount of poloxamer 188 is about 0.1%, or about 0.43%, or about 0.5%, or about 1%, or about 2%, or about 2.5%, or about 3% w/w.

In one embodiment, the pharmaceutical composition further comprises the diluent selected from the group consisting of microcrystalline cellulose, calcium hydrogen phosphate, cellulose, compressible sugar, dibasic calcium phosphate dehydrate, lactose, lactose monohydrate, mannitol, starch, tribasic calcium phosphate, and their combinations. In some embodiments, the weight content of the diluent in the pharmaceutical composition is about 1-40%w/w.

In another embodiment, the pharmaceutical composition contains microcrystalline cellulose, wherein the amount of microcrystalline cellulose is from about 1% to about 40% w/w, or from about 1% to about 35% w/w, or from about 1% to about 25% w/w, or from about 5% to about 25% w/w, or from about 10% to about 25% w/w, or from about 15% to about 25% w/w. In particular embodiments, the amount of microcrystalline cellulose is about 5% w/w, or about 10% w/w, or about 15% w/w, or about 19.46% w/w, or about 20% w/w, or about 21% w/w, or about 22% w/w, or about 23% w/w, or about 24% w/w, or about 24.04% w/w, or about 25% w/w, or about 30% w/w, or about 35% w/w, or about 40% w/w.

In one embodiment, the pharmaceutical composition comprises disintegrant selected from the group consisting of sodium carboxymethyl starch, low-substituted hydroxypropyl cellulose, croscarmellose sodium, crospovidone, microcrystalline cellulose, modified corn starch, povidone, pregelatinized starch, and their combinations. In some embodiments, the weight content of the disintegrant in the pharmaceutical composition is about 1-20% w/w.

In another embodiment, the pharmaceutical composition contains low-substituted hydroxypropyl cellulose, wherein the amount of low-substituted hydroxypropyl cellulose is from about 0.1% to about 3% w/w, or from about 0.1% to about 2.5% w/w, or from about 0.5% to about 3% w/w, or from about 0.5% to about 2.5% w/w, or from about 0.5% to about 2% w/w, or from about 1% to about 3% w/w, or from about 1% to about 2% w/w. In particular embodiment, the amount of low-substituted hydroxypropyl is about 0.1%, or about 0.43%, or about 0.5%, or about 1%, or about 2%, or about 2.29%, or about 2.5%, or about 3% w/w, or about 3.5%, or about 4% w/w.

In one embodiment, the pharmaceutical composition contains sodium carboxymethyl starch, the amount of sodium carboxymethyl starch is from about 1% to about 20% w/w, or from about 1% to about 15% w/w, or from about 1% to about 10% w/w, or from about 1% to about 8% w/w, or from about 2% to about 8% w/w. In particular embodiment, the amount of sodium carboxymethyl starch is about 1%, or about 2%, or about 3%, or about 4%, or about 4.86%, or about 5%, or about 6%, or about 7% w/w, or about 8%, or about 9%, or about 10%, or about 13%, or about 15% w/w.

In one embodiment, the pharmaceutical composition contains pregelatinized starch, the amount of pregelatinized starch is from about 1% to about 20% w/w, or from about 1% to about 15% w/w, or from about 1% to about 10% w/w, or from about 5% to about 9% w/w. In particular embodiment, the amount of pregelatinized starch is about 1%, or about 2%, or about 3%, or about 4%, or about 5%, or about 6%, or about 7% w/w, or about 8%, or about 8.27%, or about 9%, or about 10%, or about 13%, or about 15% w/w.

In one embodiment, the pharmaceutical composition further comprises binder selected from the group consisting of povidone, copovidone, hydroxypropyl cellulose, low-substituted hydroxypropyl cellulose, hydroxypropyl methyl cellulose, ethyl cellulose and pregelatinized starch, and their combinations. In some embodiments, the weight content of the binder in the pharmaceutical composition is about 1-20%w/w.

In one embodiment, the pharmaceutical composition further contains copovidone, the amount of copovidone is from about 1% to about 20% w/w, or from about 1% to about 15% w/w, or from about 1% to about 10% w/w, or from about 1% to about 8% w/w, or from about 2% to about 5% w/w. In particular embodiment, the amount of copovidone is about 1%, or about 2%, or about 3%, or about 3.80%, or about 4%, or about 5%, or about 10%, or about 15% w/w.

In one embodiment, the pharmaceutical composition further comprises lubricant selected from the group of consisting of magnesium stearate, calcium stearate, polyvinyl alcohol, sodium stearyl fumarate, stearic acid, talc, and their combinations. In some embodiments, the weight content of the lubricant in the pharmaceutical composition is about 0.1-5%w/w.

In one embodiment, the pharmaceutical composition contains magnesium stearate, the amount of magnesium stearate is from about 0.1% to about 5% w/w, or from about 0.1% to about 4% w/w, or from about 0.1% to about 3% w/w, or from about 0.1% to about 2.5% w/w, or from about 0.5% to about 3% w/w, or from about 0.5% to about 2.5% w/w, or from about 0.5% to about 2% w/w, or from about 1% to about 3% w/w, or from about 1% to about 2% w/w. In particular embodiment, the amount of magnesium stearate is about 0.1%, or about 0.42%, or about 0.5%, or about 1%, or about 2%, or about 2.5%, or about 3%, or about 3.5%, or about 4%, or about 4.5%, or about 5% w/w.

In other embodiments, the pharmaceutical composition further comprises glidant selected from the group of consisting of colloidal silicon dioxide, talc, starch, starch derivatives, and their combinations. In some embodiments, the weight content of the glidant in the pharmaceutical composition is about 0.1-5%w/w.

In other embodiments, the pharmaceutical composition contains colloidal silicon dioxide, the amount of colloidal silicon dioxide is from about 0.1% to about 5% w/w, or from about 0.1% to about 4.5% w/w, or from about 0.1% to about 4% w/w, or from about 0.5% to about 5.0% w/w, or from about 0.5% to about 3% w/w, or from about 0.5% to about 2% w/w, or from about 0.5% to about 1.5% w/w. In particular embodiment, the amount of colloidal silicon dioxide is about 0.1% w/w, or about 0.5% w/w, or about 0.75% w/w, or about 1.25% w/w, or about 1.5% w/w, or about 2% w/w.

In one embodiment, the pharmaceutical composition comprises a) about 30% to about 50% w/w nitazoxanide and b) about 30% to about 50% w/w atovaquone. In a related embodiment, the composition comprises a) about 33% w/w nitazoxanide and b) about 33% w/w atovaquone. In another related embodiment, the composition further comprises a) about 0.5% to about 2% w/w SLS, b) about 15% to about 25% w/w microcrystalline cellulose, c) about 1% to about 10% w/w sodium carboxymethyl starch, d) about 3% to about 4% w/w povidone, and e) about 0.1% to about 3% w/w magnesium stearate. **In** another embodiment, the pharmaceutical composition comprises a) about 33% w/w nitazoxanide, b) about 33% w/w atovaquone, c) about 1% w/w SLS, d) about 23% w/w microcrystalline cellulose, e) about 5% w/w sodium carboxymethyl starch, f) about 4% w/w povidone, and g) about 1% w/w magnesium stearate.

In another embodiment, the pharmaceutical composition comprises a) about 30% to about 50% w/w nitazoxanide and b) about 30% to about 50% w/w atovaquone. **In** a related embodiment, the composition comprises a) about 35% w/w nitazoxanide and b) about 35% w/w atovaquone. **In** another related embodiment, the composition further comprises a) about 0.5% to about 2% w/w poloxamer 188, b) about 15% to about 25% w/w microcrystalline cellulose, c) about 1% to about 10% w/w sodium carboxymethyl starch, d) about 3% to about 4% w/w povidone, and e) about 0.1% to about 3% w/w magnesium stearate. In another embodiment, the pharmaceutical composition comprises a) about 35.11% w/w nitazoxanide, b) about 35.11% w/w atovaquone, c) about 0.5% w/w poloxamer 188, d) about 20% w/w microcrystalline cellulose, e) about 5.18% w/w sodium carboxymethyl starch, f) about 3.6% w/w povidone, and g) about 0.5% w/w magnesium stearate.

In one embodiment, the pharmaceutical composition comprises a) about 15.86% w/w nitazoxanide, b) about 15.86% w/w atovaquone, c) about 4.71% w/w ribavirin, d) about 19.46% w/w microcrystalline cellulose PH101, e) about 0.43% w/w poloxamer 188, f) about 24.04% w/w microcrystalline cellulose PH102, g) about 4.86% w/w sodium carboxymethyl starch, h) about 3.80% w/w copovidone, i) about 0.42% w/w magnesium stearate, j) about 8.27% w/w pregelatinized starch, k) about 2.29% low-substituted hydroxypropyl cellulose.

The chemical name of Nitazoxanide (NTZ) is: o-[N-(5-nitro-1,3-thiazol-2-yl)carbamoyl]phenol acetate), CAS: 55981-09-4, molecular formula: C₁₂H₉N₃O₅S, molecular weight: 307.28, and its chemical formula is as follows:

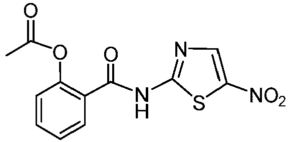

The chemical name of Tizoxanide (TIZ) is: 2-Hydroxy-N-(5-nitro-2-thiazolyl)benzamide, CAS: 1246817-56-0, molecular formula: C₁₀H₇N₃O₄S, molecular weight: 265.24528, and its chemical formula is as follows:

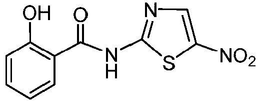

The chemical name of Atovaquone (ATQ) is: 2-(trans-4-(4-chlorophenyl) cyclohexyl)-3-hydroxy-1,4-naphthalenedione, CAS: 95233-18-4, molecular formula: C₂₂H₁₉ClO₃, molecular weight: 366.84, and its chemical formula is as follows:

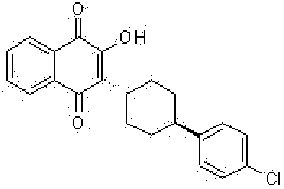

The chemical name of Ribavirin is: 1-[(*2R*, *3R, 4S*, *5R*)-3,4-dihydroxy-5-(hydroxymethyl)Oxolane-2-yl]-1H-1,2,4-triazole-3-carboxamide, also known as 1-(β-D-ribofuranosyl)-1H-1,2,4-triazole-3-carboxamide, CAS number: 36791-04-5, molecular formula: C₈H₁₂N₄O₅, molecular weight: 244.21, and its chemical formula is as follows:

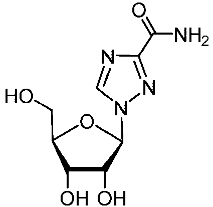

The present invention provides a new pharmaceutical composition of nitazoxanide, atovaquone and/or ribavirin for inhibiting coronavirus infection.

The present invention also provides nitazoxanide, atovaquone and/or ribavirin in combination for synergistic inhibition of coronavirus infections, such as SARS-CoV-2 infection and its mutant strains infection.

The above studies provide at least one novel pharmaceutical composition or at least one new available therapy regimen for the treatment of SARS-CoV-2 or other coronavirus infections and associated diseases or conditions thereof, such as pneumonia, that can achieve effective treatment of the virus infection, or rapid alleviation of conditions associated thereof, or reduction in the duration of clinical symptoms, as well as diminishing the potential for virus infection and retransmission.

In one embodiment, the pharmaceutical composition or combinatorial drugs disclosed by the present invention show no new adverse events, and have similar safety profiles compared to each drug of the combinatorial drugs when used alone, and there are no negative drug-drug interactions.

In one embodiment, the pharmaceutical composition and combinatorial drugs disclosed by the present invention are used to relieve at least one symptom of the associated diseases or conditions, non-limiting examples of such symptoms including those selected from cough, decreased appetite, asthma, dyspnea, fever, general malaise, dizziness, nausea, and pneumonia.

In the further embodiment, the pharmaceutical composition or combinatorial drugs disclosed in the present invention also has the potential to reduce the occurrence of cytokine storm, it is expected to antagonize the cytokine storm of the patient, reduce the inflammatory response, and decrease the damage of the inflammatory response to the patient's lung tissue and multiple organs.

The pharmaceutical composition or combinatorial drugs regimen provided by the present invention refers to the treatment or prevention of coronavirus infection, including nitazoxanide or tizoxanide, and ribavirin and/or atovaquone, especially the pharmaceutical composition comprising tizoxanide, ribavirin and atovaquone (hereinafter referred to as the pharmaceutical composition of the present invention, or the pharmaceutical composition **Anticov-1**), compared with the existing novel coronavirus treatment drugs, it is the only specific treatment drug for novel coronaviruses that has the following four important characteristics at the same time, including: convenient oral administration, guaranteed safety, strong antiviral effect and the antiviral mechanism is not affected by virus mutation, and highly effective anti-inflammatory storm. These features of the pharmaceutical composition provided by the present invention have great potential for the control of the novel coronavirus epidemic situation, Other novel coronavirus treatment drugs, including Merck's Molnupiravir and Pfizer's Paxlovid (composed of PF-07321332 and ritonavir) anti-coronavirus drugs, only have some of the above characteristics. Therefore, the pharmaceutical composition provided by the present invention is expected to cover the whole process and the whole population from the prevention of high-risk groups after close contact, the progression of mild and moderate to severe disease, and the treatment of severe and critically ill patients, and it is expected to contribute to the control of the novel coronavirus global pandemic.

First, convenient oral administration. People infected with the novel coronavirus or high-risk close contacts, such as family members of confirmed cases, neighbors in the community, colleagues in the unit, etc., only need to take medicine by themselves, and do not need to go to the hospital for injection medicine, reduce the risk of transmission and fear, reduce medical costs, and people can maintain normal social activities with simple protection. The pharmaceutical composition of the invention is a tablet, which is easy to carry and take, and can be used by both symptomatic patients and asymptomatic infected patients. Novel coronavirus cases and high-risk close contacts can take drugs in home isolation or designated isolation places to achieve the effect of infection prevention and control. In addition, the production capacity and cost of oral drugs also have advantages, which are of great significance to the control of the epidemic.

Second, guaranteed safety. From the perspective of mechanism of action, most oral drugs for novel coronaviruses target the viral RNA replication process, or cause mutations in viral genes. The long-term safety data of this class of drugs, especially the risk of genotoxicity and promoting new virus variants, need attention. Studies have reported that Merck's anti-coronavirus drugs can be integrated into the genetic material of mammalian cells, causing mutations during cell replication, theoretically there is a risk of further inducing cancer and neonatal deformities (J. Biol. Chem. (2021) 297 100867). The 3CL protease inhibitor has not been approved for any indication before, and the long-term safety data is unknown. The three component drugs contained in the novel specific anti-coronavirus pharmaceutical composition provided by the present invention have been approved by regulatory authorities such as the FDA, and have been used clinically around the world for decades, and are safely used for hundreds of thousands or even more patients around the world, so clinical safety is guaranteed. Based on the pre-clinical significant anti-novel coronavirus activity data, the dose of the pharmaceutical composition of the present invention for the treatment of novel coronavirus is much lower than the drug dose approved by FDA. For example, the usual clinical dosage of the pharmaceutical composition provided by the present invention (Anticov-1), for the treatment or prevention of infection by novel coronavirus and its mutant strains is: the active ingredient NTZ is about 999 mg/day, only 1/4 of the maximum administered dosage (MAD) of NTZ used safely in clinical practice, and not higher than the usual clinical dose; The active ingredient RBV is about 297 mg/day, which is 1/8 of the clinically safe maximum administered dose (MAD) of RBV, and only 1/4 of the clinically commonly used dose; the active ingredient ATQ is about 999 mg/day, which is 1/3 of the maximum administered dosage (MAD) of ATQ used safely in clinical practice, and only 2/3 of the clinically commonly used dose. The pharmaceutical composition provided by the present invention can effectively combat novel coronaviruses while minimizing the safety risks. Facing the treatment needs of tens of millions or even hundreds of millions of people, it is a very important advantage to fully guarantee the safety of therapeutic or preventive drugs. A drug whose safety has been proven over time will make patients more at ease in treatment and more confident in defeating the epidemic.

In addition, due to the high plasma protein binding rate of atovaquone and nitazoxanide, both about 99% (refer to Beerahee M. Clinical pharmacology of atovaquone and proguanil hydrochloride. [J]. Journal of Travel Medicine, 1999, 6 suppl 1:S13-7*.; Alinia^{™} PRESCRIBING INFORMATION-FDA*.). The present application also studied and compared the in vivo plasma protein binding rate of atovaquone and nitazoxanide combined administration and separate administration. The results demonstrate that the administration of atovaquone combined with nitazoxanide has no significant difference in plasma protein binding compared with separate administration (the average plasma protein binding rates of atovaquone and nitazoxanide in the combined administration group are 99.86% and 99.84%, respectively, and the average plasma protein binding rates of atovaquone and nitazoxanide in the separate administration group are 99.84% and 98.95%), that is, there is no competition between the atovaquone and nitazoxanide for binding to plasma proteins. Compared with alone administration, combined administration will not affect the distribution and elimination of the two drugs in vivo, and competition for plasma protein binding site by one drug will not result in an increase in the free form of another drug. The safety of the pharmaceutical composition or combinatorial drugs regimen involved in this application is further proved.

Third, it has strong antiviral effect and the antiviral mechanism is not affected by virus mutation. WHO has designated the novel coronavirus Alpha, Beta, Gamma, Delta and Omicron mutants as "Variant of Concern". The new mutant strains have mutations at some key sites of the spike protein used to recognize cell receptors and invade cells, resulting in increased the viral load, virus transmission ability and the risk of reinfection, which is an important reason for the recurrence of global epidemics. At the same time, new mutated strains may also lead to reduced vaccine efficacy and reduced effectiveness of neutralizing antibodies. For example, due to the insufficient effectiveness of treating novel coronavirus mutant strains, the FDA urgently suspended the use of Lilly's neutralizing antibody drugs in June 2021, after resumption, use is only allowed in areas where the prevalence of drug-resistant strains is less than 5%. The pharmaceutical composition of the present invention has a broad-spectrum antiviral activity mechanism, and the antiviral purpose is mainly achieved by activating the natural antiviral genes and natural antiviral immune barriers of body cells, so its antiviral mechanism of action is not affected by virus variation.

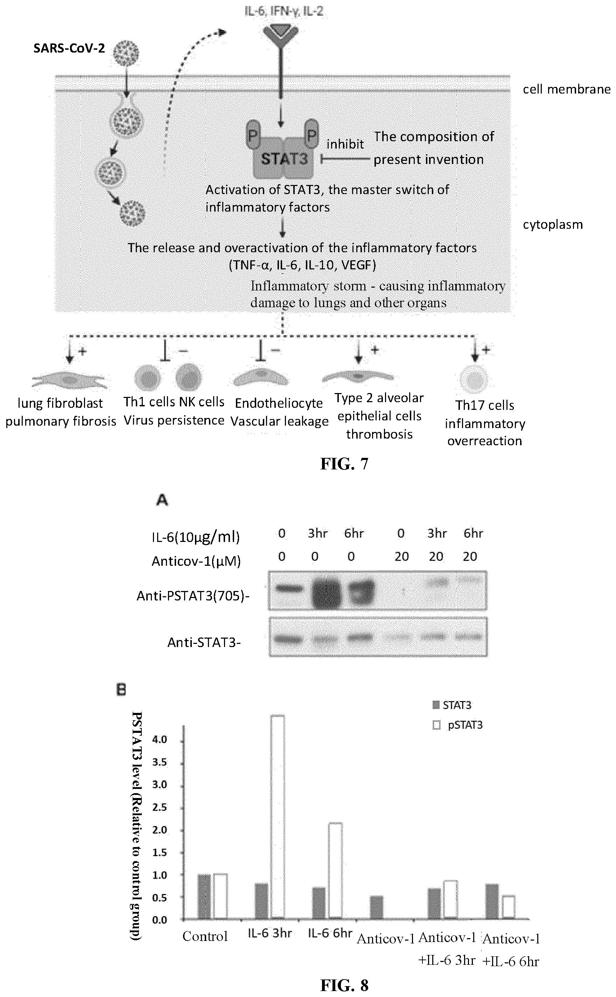

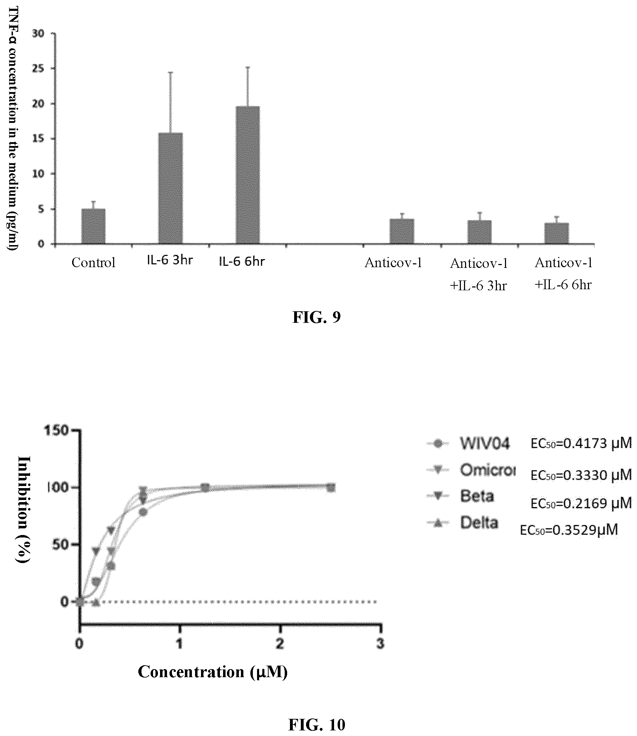

Fourth, high anti-inflammatory storm effect. Inflammatory storm, also known as cytokine storm, is an overreaction of the body's immune system against the novel coronavirus. A massive release of cytokines in the body, resulting in an acute inflammatory response and organ damage, which is the key factor for the transformation of novel coronavirus pneumonia from mild/moderate to severe, critical and even death. In the body's cells, the "master switch" that controls inflammatory factors is STAT3, since the outbreak of the novel coronavirus, many scientific papers have reported that STAT3 acts as a master switch to control the inflammatory storm in the pathological mechanism of the novel coronavirus (Microbial Pathogenesis 154 (2021) 104836, Cell Death & Differentiation (2020) 27:3209-3225). STAT3 is involved in the induction of inflammatory response during novel coronavirus infection, inhibits antiviral interferon response, and leads to imbalance of antiviral adaptive immune response (FIG. 7). Therefore, inhibiting the activation of the STAT3 pathway is a powerful strategy to treat the novel coronavirus infection from the source switch of the inflammatory storm. The pharmaceutical composition of the present invention can inhibit the activation of the STAT3 which is the "master switch" of inflammatory factors and inhibit the release of inflammatory factors (such as TNF-α), to reduce the inflammatory response (**FIG. 8** **and** **FIG. 9**) and prevent the outbreak of the inflammatory storm. Its anti-inflammatory storm effect is a heavy weapon for the treatment of novel coronavirus pneumonia and the prevention of novel coronavirus infection from causing disease in the body. It is expected to inhibit inflammatory cytokines in patients with novel coronavirus pneumonia and reduce inflammatory damage in the lungs and other organs, and this is also the only drug in development on the market that not only inhibits the novel coronavirus itself, but also acts on the impact of the inflammatory storm on the course of the disease. The pharmaceutical composition of the present invention can be used simultaneously to prevent the conversion of mild and moderate to severe disease, and to treat severe and critically ill patients.

To sum up, the pharmaceutical composition of the present invention is the only innovative novel specific anti-coronavirus drug in developing all over the world that has four advantages (convenient oral administration, guaranteed safety, strong antiviral effect and the antiviral mechanism is not affected by virus mutation, and highly effective anti-inflammatory storm) at the same time. The pharmaceutical composition of the present invention is expected to cover the whole process and the whole population from the prevention of high-risk groups after close contact, the progression of mild and moderate to severe disease, and the treatment of severe and critically ill patients, and avoid the implementation restrictions that other drugs such as nucleosides and protease inhibitors may face, which is conducive to the global promotion and use and is expected to contribute to the control of the global pandemic of the novel coronavirus.

Also described is a method for treating or preventing coronavirus infection in a patient or diseases or conditions associated thereof by administering the new pharmaceutical composition of the co-preparation or the combinatorial drugs of the present invention. In certain embodiments, the prevention of diseases or conditions caused by or associated with coronavirus infection includes chemoprevention or drug prevention. The coronavirus is SARS-CoV-2 or its mutant strains, SARS, MERS, 229E, NL63, OC43 or HKU1. In one embodiment, the associated disease or condition is pneumonia. The method relieves at least one symptom of the diseases or conditions, wherein the non-limiting examples of at least one symptom selected from the group consisting of cough, decreased appetite, asthma, dyspnea, fever, general malaise, dizziness, nausea, diminished or lost sense of smell, and pneumonia.

In some embodiments, the pharmaceutical composition for use in a method of the treatment or prevention of diseases or conditions caused by a coronavirus infection or associated with it, comprising administering the pharmaceutical composition 1-4 times a day to a subject in need thereof in 1-5unit formulations each time. In some embodiments, the method comprises administering the pharmaceutical combination 2-4 times per day to a subject in need thereof, in 2-4unit formulations each. In some embodiments, the subject in need thereof are mild patients, common patients, severe patients, or critical patients caused by coronavirus infection. In some embodiments, the method further comprises simultaneously or sequentially administering to a subject in need thereof a therapeutically effective amount of at least one other therapeutic agent, wherein the other therapeutic agents are antiviral antibody medicines, or other antiviral medicines, or other symptomatic treatment medicines for symptoms caused by coronavirus infection. In some embodiments, the coronavirus is SARS-CoV-2 or its mutant strains, SARS, MERS, 229E, NL63, OC43 and/or HKU1, especially SARS-CoV-2 or its mutant strains. In some embodiments, mutant strains of SARS-CoV-2 include, but are not limited to Alpha (B.1.1.7), Beta (B.1.351), Gamma (P.1), Delta (B.1.617.2), Epsilon (B.1.427/B.1.429), Zeta (P.2), Eta (B.1.525), Theta (P.3), Iota (B.1.526), Kappa (B.1.617.1), Lambda (C.37), Mu(B.1.621) and Omicron (B.1.1.529 and its descendants lineages BA.1, BA.1.1, BA2, BA.2.12.1, BA.2.75, BA.3, BA.4, BA.4.6, BA.5, XE). In some embodiments, the diseases or conditions caused by or associated with the coronavirus infection is pneumonia (COVID-19) caused by SARS-CoV-2 infection and mutant strains thereof infection.

### IV. Examples

### Example 1: Cell culture

Vero E6 African green monkey kidney cell line (ATCC, No.1586) was obtained from American Type Culture Collection and subcultured in DMEM medium (Gibco Invitrogen) supplemented with 1% (v/v) penicillin + streptomycin at 37 °C in an atmosphere of 5% CO2.

### Example 2: Isolation of virus strain

The original strain of SARS-CoV-2 (hCoV-19/China/CAS-B001/2020) is from the Institute of Microbiology, Chinese Academy of Sciences. Another original strain of SARS-CoV-2, WIV04 (nCoV-2019BetaCoV/Wuhan/WIV04/2019), is from the Wuhan Institute of Virology, Chinese Academy of Sciences. The SARS-CoV-2 Beta mutant strain was obtained from the National Pathogen Resource Center (NPRC) (2.062100001). The SARS-CoV-2 Delta mutant strain was obtained from NPRC (China Science and Technology Resource. 16698.06. NPRC 6. CCPM-B-V-049-2105-8). SARS-CoV-2 Omicron mutant strain was obtained from Wuhan Institute of Virology, Chinese Academy of Sciences. Different mutant strain were cultured in Vero E6 cells, and the virus titer was determined by measuring 50% tissue culture infective dose (TCID₅₀) by immunofluorescence assay. The full name of "TCID₅₀" is median tissue culture infective dose, tissue median infective dose, or 50% tissue cell infective dose, which refers to the amount of virus required to cause half of the cytopathic effect (CPE) or half of the cell death in the culture plate well or test tube, and is used to characterize the titer of the virus. All the infection experiments were performed in a Biosafety Level 3 (BLS-3) laboratory.

Experimental drug: Nitazoxanide (NTZ) API was purchased from Zhejiang Cheng Yi Pharmaceutical Co., Ltd. (Lot No. 0501-2020-03901), ribavirin (RBV) API was purchased from AZICO Biophore India Pvt. Ltd (Lot No. 4038/3/006/21), and atovaquone (ATQ) API was purchased from NURAY Chemicals Pvt. Ltd (Lot No. ATORD210001). "**Anticov-1**" is the pharmaceutical composition containing active ingredients NTZ, ATQ and RBV.

### Example 3: Antiviral activity test of drug

In order to evaluate the antiviral efficacy of the pharmaceutical composition of the present invention, the cells were pretreated with different concentrations of the anti-coronavirus drug of the present invention for 8 hours or overnight, and DMSO was set as a negative control at the same time. The virus was then added to infect Vero E6 cells with a total of 100 TCID₅₀ (TCID₅₀=10^{7.0}/mL) of SARS-CoV-2. After 72 hours of infection, the cytopathic effect (CPE) was observed. The virus-drug mixture is then removed and the cells are further cultured with fresh medium containing the drug. Cell supernatants were collected after incubation 48 hours and lysed in lysis buffer (Takara, Cat. No. 9766) for quantitative analysis.

According to the kit instructions, 100 µL cell culture supernatant was collected, and the viral RNA was extracted using the MiniBEST Viral RNA/DNA Extraction Kit (Takara, Cat. No. 9766). RNA was eluted in 30 µL RNase-free water. Use the reverse transcription kit (Takara, article number RR047A) that has gDNA Eraser to carry out reverse transcription, and qRT-PCR was performed on StepOne Plus Real-time PCR System (Applied Biosystem) with TB Green Premix Ex Taq II (Takara, Cat. No. RR820A). GraphPad Prism 6 software was used to plot the dose-response curve of viral RNA copies relative to drug concentration.

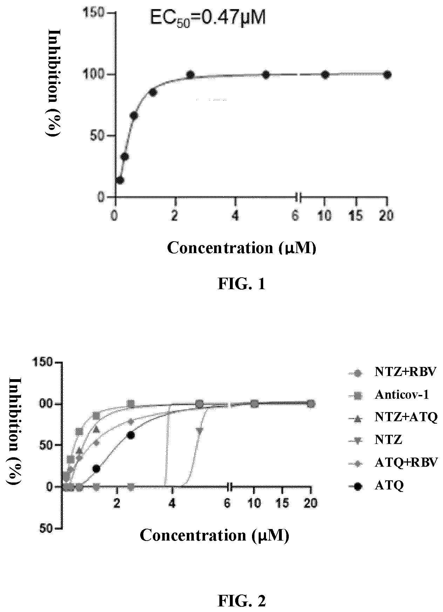

The results of the dose-response curve of the SARS-CoV-2 virus inhibition rate relative to the drug concentration are shown in **FIG. 1****,** and the anti-coronavirus inhibitory activity EC₅₀ of Anticov-1 is 0.47 µM.

### Example 4: Synergistic effect of anti-SARS-CoV-2 virus activity of Anticov-1

The wild strain of SARS-Cov-2 (hCoV-19/China/CAS-B001/2020 strain, TCID50=107.0/mL) was used to infect Vero-E6 cells. Six different experimental drug combinations (Anticov-1, NTZ+RBV, NTZ+ATQ, ATQ+RBV, NTZ, ATQ) were added to the 96-well plate at concentrations of 20µM, 10µM, 5µM, 2.5µM, 1.25µM, 0.625µM, 0.31µM, 0.16µM (solvent DMSO) respectively, and the DMSO solvent without drug was used as a blank control. The cells were incubated in an incubator for 2 hours. After the incubation, the drug was aspirated, and 100 µL wild strain of SARS-CoV-2 (hCoV-19/China/CAS-B001/2020) dilution (100 TCID₅₀ in total) was added to the wells except the negative control, the negative control added with virus-free DMEM medium, and cultured in an incubator for 2 hours. The virus solution was aspirated and removed and the new drug dilution was re-added. The cell plate is put into an incubator and cultivated for 72 hours to observe the cytopathic effect (CPE), and the EC₅₀ value was calculated according to the observation results of CPE (results are shown in **Table 1**), and the inhibition rates at the dilution concentration points were fitted with curves respectively, as shown in **FIG. 2**.

**Table 1: EC₅₀ of 6 test products**

| Experimental drug | EC₅₀ (µM) |
|---|---|
| Anticov-1 | 0.4724 |
| NTZ+RBV | 3.814 |
| NTZ+ATQ | 0.7259 |
| ATQ+RBV | 1.243 |
| NTZ | 4.901 |
| ATQ | 2.04 |

The results in **FIG. 2** and **Table 1** show that the combination of NTZ+ATQ shows a significant synergistic anti-SARS-CoV-2 virus effect. What is even more surprising is that, compared with any two-drug combination regimen or mono therapy regimen, **Anticov-1,** a three-drug combination of NTZ, ATQ and RBV, shows an extremely surprising and significant synergistic antiviral effect on SARS-CoV-2, excellent EC₅₀ levels were achieved, and the antiviral synergistic effect was significantly better than other combinatorial drug regimens.

**Example 5: cytotoxicity research of Anticov-1.**

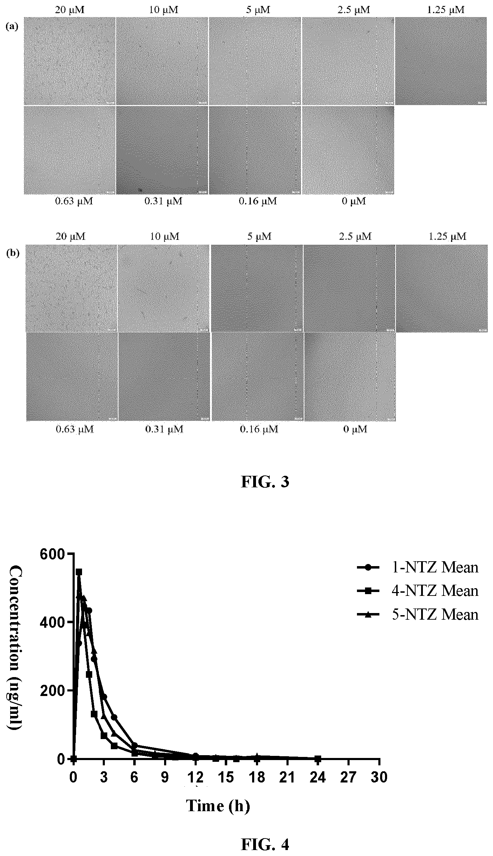

Vero E6 cells were seeded into 96-well plates and cultured in a 5% CO2/37°C incubator for 24 hours. Anticov-1 was added to the 96-well plate at concentrations of 20 µM, 10 µM, 5 µM, 2.5 µM, 1.25 µM, 0.63 µM, 0.31 µM and 0.16 µM respectively, and DMEM medium was added as a control well. At the same time, the wells added with DMEM medium but not seeded with cells were used as blank wells. After 24 hours and 48 hours, the cytotoxic effect (Cytotoxic Effect) on Vero E6 cells at each concentration was observed respectively (results are shown in **FIG. 3(a), 3(b)** respectively). The results show that after 24 or 48 hours incubation, no obvious cytotoxicity was observed for **Anticov-1** test product at a concentration of at least 5 µM and lower, which exhibits the CC₅₀ of **Anticov-1** in Vero E6 cells is ≥5µM.

After 72 hours of incubation, the Cytostatic Effect of **Anticov-1** on Vero E6 cells was tested. 10 µL CCK-8 solution was added to the 96-well plate, and the O.D. value was measured by using the microplate reader at the main wavelength of 450 nm after 1 hour. the cell inhibition ratio of **Anticov-1** at each concentration was calculated, and the AP₅₀ (concentration for 50% Antiproliferation effect) of Anticov-1 in Vero E6 cells is 1.988 µM.

### Example 6: PK of Anticov-1 tablet in rats

Experimental drug Anticov-1 tablet specification: NTZ 111 mg/ATQ 111 mg/RBV 33 mg per tablet.

Equivalent dose in rats was converted according to the clinical dose in human, and 5 experimental dose groups were designed (see **Table 2** for details). Among them, group 4 was converted according to the clinical administration of 3 tablets, and the remaining experimental groups were converted according to the clinical administration of 4 tablets.

**Table 2 Dosage of different experimental groups**

| Group | Dosage of administration |
|---|---|
| Group 1 | Nitazoxanide (NTZ, 46.6 mg/kg) |
| Group 2 | Atovaquone (ATQ, 46.6 mg/kg) |
| Group 3 | Ribavirin (RBV, 13.9 mg/kg) |
| Group 4 | NTZ (QX1A,35.0 mg/kg) + ATQ (QX1C, 35.0 mg/kg) + RBV (QX1B, 10.4 mg/kg) |
| Group 5 | NTZ (QX1A,46.6 mg/kg) +ATQ (QX1C, 46.6 mg/kg) +RBV (QX1B,13.9 mg/kg) |

A single dose was administered to healthy SD rats, and blood was taken according to the blood collection plan. After sample processing, analysis, and data processing, the plasma concentration-time curves of each group were drawn respectively. The pharmacokinetic parameters of each group of medicines in rats was calculate by WinNonlin 6.1 software.

The change of NTZ plasma concentration after single administration in group 1, group 4 and group 5 is shown in **FIG. 4**. There is no obvious difference between NTZ plasma concentration of NTZ administered alone (group 1) at a dose of 46.6 mg/kg and that of NTZ administered in combination at the same dose (group 5). There is no significant difference in the overall change trend of plasma concentration over time.

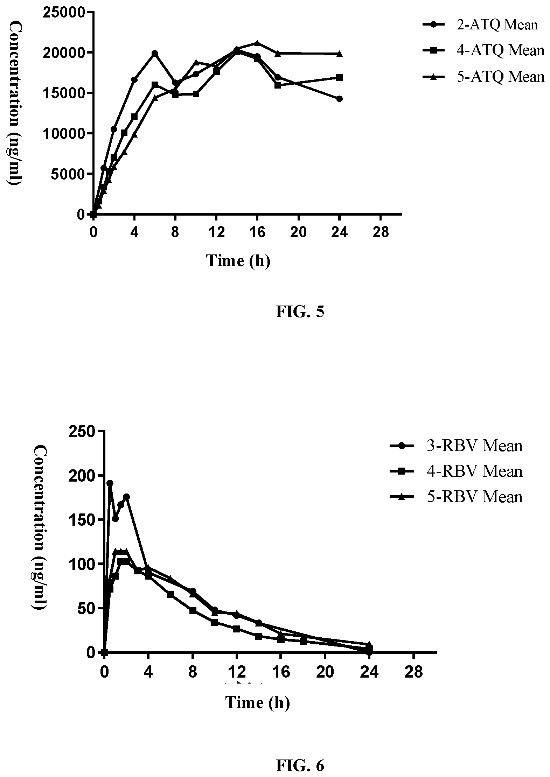

The change of ATQ plasma concentration after single administration in group 2, group 4 and group 5 is shown in **FIG. 5**. There is no significant difference in the overall change trend of plasma concentration over time, among which ATQ plasma concentration shows a downward trend at 16-24 hours after ATQ administered alone, while ATQ plasma concentration shows a flat or rising trend at 16-24 hours after combined administration. In all experimental groups, the exposure level (AUC₀₋₂₄ₕ) of ATQ administered alone is similar to the exposure level administered in combination at the same dose, and the exposure level is decreased with the combined administration dose decrease.

The change of RBV plasma concentration after single administration in group 3, group 4 and group 5 is shown in **FIG. 6**. Compared with the results of nitazoxanide and atovaquone, within 0.5-4 hours after administration, the RBV plasma concentration administered alone at 13.9 mg/kg (group 3) is greater than that administered in combination at the same dose (group 5) and also greater than that administered in combination at 10.4 mg/kg (group 4). In all experimental groups, the exposure level (AUC₀₋₂₄ₕ) of RBV administered alone is slightly greater than that of combined administration at the same dose, and the exposure level further decrease with the dose of combined administration decreased.

Overall, the results of the in vivo PK experimental in rats shows that the three-drug combination regimen has little effect on the PK of each active ingredient administered alone, and the plasma concentration levels of each drug are almost the same after at least 4 hours of single administration, and the combination administration even has an advantage on maintaining the plasma concentration of a certain drug (such as ATQ) after 16 hours compared with mono therapy.

To evaluate the effectiveness of an antiviral drug, it is also mainly measured by comparing the drug's virus-inhibiting activity (EC₅₀) and whether the plasma concentration can reach EC₅₀. If the plasma concentration is much higher than EC₅₀, it means that the antiviral clinical effect of the drug can be expected; If the plasma concentration of the drug cannot reach the EC₅₀, no matter how low the EC₅₀ value is, it has no clinical significance. Compared with the anti-novel coronavirus drugs currently under development or approved (Pfizer's Paxlovid and Merck's Molnupiravir), **Anticov-1** has many advantages. The obvious advantages in the effect of anti-novel coronavirus are shown in **Table 3** below. The active ingredient of the anti-coronavirus drug of the present invention has good pharmacokinetic characteristics, and after taking the drug, the plasma concentration can continue to exceed the effective dose of novel coronavirus inhibition by dozens of times, much higher than Paxlovid or Molnupiravir.

**Table 3. Comparison of anti-SARS-CoV-2 activity between Anticov-1 and other drugs**

| Drug Name | EC₅₀ (µM) | Plasma concentration (µM) | Plasma concentration/EC₅₀ |
|---|---|---|---|
| Paxlovid^{a} | 0.074 - 4.48* | > 1.13 | >15.3 |
| Molnupiravir^{b} | 0.32 - 2.03 | 2.09 | 1.03 - 6.53 |
| **Anti-coronavirus drug of the present invention** | **0.47** | **> 30** | **>63.8** |

| | | | |
|---|---|---|---|
| a. Owen et al., Science, 2021 ; *The EC₅₀ of protease inhibitor PF-07321332 combined with p-glycoprotein inhibitor CP-100356 is 0.074 µM, and the EC₅₀ of PF-07321332 administrated alone is 4.48 µM b. Summary of Product Characteristics for Lagevrio | | | |

### Example 7: Anti-inflammatory test: blocking IL-6-mediated activation of STAT3 in human blood cells

Anticov-1 was co-incubated with human myeloma cells U266, and IL-6 was used to induce excessive inflammation, and the expression of pSTAT3 and STAT3 was measured.

The test results are shown in **FIG. 8****,** the phosphorylated STAT3 (pSTAT3) level of U266 cells treated with **Anticov-1** is equal to or lower than that of the control group not induced by IL-6; Compared with the expression level of pSTAT3 in U266 cells directly induced by IL-6 without **Anticov-1** treatment, the expression level of pSTAT3 in the former is only about a quarter of that in the latter after 3 hours and 6 hours. The results suggest that **Anticov-1** can significantly inhibit the activation of STAT3, thereby inhibiting the inflammatory storm.

### Example 8: Anti-inflammation test: block the release of TNF-α in human blood cells mediated by IL-6

After Anticov-1 was co-incubated with human myeloma cells U266, IL-6 was used to induce excessive inflammation, and the expression of TNF-α was determined.

The test results are shown in **FIG. 9****.** The expression of TNF-α in the cells treated with **Anticov-1** for 2 hours has no significantly different from, or even lower than, that of the control group without IL-6 induction. However, the expression levels of TNF-α in cells not treated with **Anticov-1** at 3 hours and 6 hours are about 3 and 4 times the expression levels of TNF-α after **Anticov-1** treatment, respectively. The results prove that **Anticov-1** can significantly inhibit the release of inflammatory factors, and can effectively inhibit the inflammatory storm.

### Example 9: Inhibitory activity of Anticov-1 against broad-spectrum novel coronavirus strains

In order to evaluate the inhibitory activity of Anticov-1 against the main epidemic novel coronavirus strains, the Vero E6 cells were seeded into 96-well plates at a density of 5000 cells/well and cultured overnight. Add Anticov-1 at concentrations of 20 µM, 10 µM, 5 µM, 2.5 µM, 1.25 µM, 0.63 µM, 0.31 µM and 0.16 µM to the 96-well plate and incubate for 2 hours respectively. Afterwards, 100 µL of the wild strain WIV04, Beta mutant, Delta mutant or Omicron mutant venom (total 100 TCID₅₀ for each strain) were added respectively to infect for 2 hours (DMEM medium was added to the blank control well without virus). After 2 hours, the virus-drug mixture was removed, and the drug-containing medium was re-added for incubation. After 72 hours, cell supernatants were collected. Cell plates were washed twice with PBS, soaked in 4% paraformaldehyde and fixed. Cell supernatants were collected, and viral RNA was extracted for reverse transcription real-time quantitative PCR (RT-qPCR). The inhibitory rate of **Anticov-1** to each SARS-CoV-2 strain at different concentrations was calculate, and the dose-response curve of the inhibitory rate and drug concentration was processed and drawn by GraphPad Prism 6 software, and the results are shown in **FIG. 10****.**

The results showed that at a concentration of 0.63 µM, Anticov-1 has an inhibitory rate of >95% against Omicron and Delta strains, and the inhibitory rates against Beta and WIV04 strains are 78.57% and 87.97%, respectively. At a concentration of 1.25 µM, Anticov-1 achieves the inhibition rate of >99% against all 4 strains. Anticov-1 has a strong inhibitory effect on all major epidemic virus strains, especially against the current major epidemic omicron, and even has a higher inhibitory activity than that in the Delta strain and wild type. It is proved again that the pharmaceutical compositions or combinatorial drugs regimen of the present invention has a broad-spectrum anti-SARS-CoV-2 specific therapeutic effect.

### Selectivity index

According to Anticov-1 test sample CC₅₀ in Vero E6 cells and the EC₅₀ of anti-4 kinds of virus strains, can calculate the selectivity index (SI) of Anticov-1 anti-4 kinds of virus strains respectively, and the result is as **Table 4.**

**Table 4: SI of Anticov-1 to 4 kinds of SARS-CoV-2 strains in Vero E6 cells.**

| Strain | CC₅₀ (µM) | EC₅₀ (µM) | SI = CC₅₀ / EC₅₀ |
|---|---|---|---|
| WIV04 | | 0.4173 | ≥11.98 |
| Omicron | ≥5 | 0.3330 | ≥15.02 |
| Delta | | 0.3529 | ≥14.17 |
| Beta | | 0.2196 | ≥22.77 |

The results show that Anticov-1 has a wide safety therapeutic index for different novel coronavirus mutant strains, suggesting that its clinical use has a wider safety range.

## Claims

1. A combinatorial drug comprising:
(i) at least one drug selected from nitazoxanide or tizoxanide or a pharmaceutically acceptable salt thereof, at least one atovaquone or a pharmaceutically acceptable salt thereof, and at least one ribavirin or a pharmaceutically acceptable salt or ester thereof;
or
(ii) at least one drug selected from nitazoxanide or tizoxanide or a pharmaceutically acceptable salt thereof, and at least one atovaquone or a pharmaceutically acceptable salt thereof.

2. The combinatorial drug of claim 1, wherein the combinatorial drug further comprises at least one antiviral antibody drug or antiviral antibody drug combination, or other antiviral drugs.

3. The combinatorial drug of claim 1 or 2 for use in a method of treating or preventing coronavirus infection or diseases or conditions associated thereof.

4. The combinatorial drug for the use of claim 3, wherein the coronavirus is selected from the group consisting of SARS-CoV-2 or its mutant strains, SARS, MERS, 229E, NL63, OC43 and HKU1, and wherein the coronavirus infection or diseases or conditions associated thereof comprises at least one of the following symptoms: cough, decreased appetite, asthma, dyspnea, fever, general malaise, dizziness, nausea, diminished or lost sense of smell, and pneumonia.

5. The combinatorial drug for the use of claim 4, wherein the coronavirus is SARS-CoV-2 or its mutant strains, and the diseases or conditions associated is pneumonia (COVID-19) caused by SARS-CoV-2 or its mutant strains infection.

6. The combinatorial drug for the use of claim 3, wherein the method comprises administration of nitazoxanide in an oral formulation, administration of atovaquone in an oral formulation, and administration of ribavirin in an oral formulation, inhalation formulation or injection formulation.

7. The combinatorial drug for the use of claim 6, wherein the method comprises administering nitazoxanide, atovaquone, and ribavirin to a subject in need thereof, wherein nitazoxanide is administered to a subject in need thereof at a total daily dose ranging from 100 mg to 3000 mg, wherein atovaquone is administered to a subject in need thereof at a total daily dose ranging from 20 mg to 3000 mg, wherein ribavirin is administered to a subject in need thereof at a total daily dose ranging from 20 mg to 2500 mg; wherein nitazoxanide, atovaquone and ribavirin can be administered once daily (QD), twice daily (BID), three times daily (TID) or four times daily (QID), respectively.

8. A pharmaceutical composition comprising an active pharmaceutical ingredient and at least one pharmaceutically acceptable excipient, carrier or diluent; wherein the active pharmaceutical ingredient comprises:
(i):
a) at least one drug selected from nitazoxanide or tizoxanide or a pharmaceutically acceptable salt thereof, b) at least one atovaquone or a pharmaceutically acceptable salt thereof, and c) at least one ribavirin or a pharmaceutically acceptable salt or ester thereof; or
(ii):
a) a therapeutically effective amount of nitazoxanide or a pharmaceutically acceptable salt thereof and
b) a therapeutically effective amount of atovaquone or a pharmaceutically acceptable salt thereof.

9. The pharmaceutical composition of claim 8 (i), wherein the active pharmaceutical ingredient comprises nitazoxanide, atovaquone and ribavirin, and wherein the weight ratio of nitazoxanide: atovaquone: ribavirin is selected from the group consisting of (0.1~10): (0.1~10): 1, 1.5:1.5:1, 2:2:1, 2.5:2.5:1, 3:3:1, 10:10:3, 3.5:3.5:1, 4:4:1, 4.5:4.5:1, 5:5:1, and any range value between any two ratios above.

10. The pharmaceutical composition of claim 9, wherein the pharmaceutical composition comprises
(i) 50 mg to 1200 mg nitazoxanide per unit formulation, 50 mg to 1200 mg atovaquone per unit formulation, and 1 mg to 500 mg ribavirin per unit formulation;
(ii) 60 mg to 600 mg nitazoxanide per unit formulation, 60 mg to 600 mg atovaquone per unit formulation, and 10 mg to 200 mg ribavirin per unit formulation; or
(iii) 111 mg nitazoxanide per unit formulation, 111 mg atovaquone per unit formulation, and 33 mg ribavirin per unit formulation.

11. The pharmaceutical composition of claim 8 (ii), wherein the active pharmaceutical ingredient consists of nitazoxanide and atovaquone in a fixed ratio.

12. The pharmaceutical composition of claim 11, wherein the fixed ratio of nitazoxanide and atovaquone is the weight ratio of nitazoxanide: atovaquone, wherein the weight ratio of nitazoxanide: atovaquone is selected from the group consisting of 1:0.1 to 1:10. 1:0.1, 1:0.2, 1:0.5, 1:0.8, 1:1, 1:1.2, 1:1.5, 1:2, 1:5, 1:10, and any range value between any two ratios above.

13. The pharmaceutical composition of claim 8, wherein the pharmaceutical composition is formulated as a tablet, capsule or suspension.

14. The pharmaceutical composition of claim 11, wherein the pharmaceutical composition comprises:
(i) 50mg to 1200 mg nitazoxanide per unit formulation, and 50 mg to 1200mg atovaquone per unit formulation;
(ii) 60mg to 500 mg nitazoxanide per unit formulation, and 100 mg to 500mg atovaquone per unit formulation; or
(iii) 165mg nitazoxanide per unit formulation, and 165mg of atovaquone per unit formulation.

15. The pharmaceutical composition of any one of claims 8-14 for use in a method of therapy, wherein the method comprises administering the pharmaceutical composition to a subject in need:
a) with 1-5 unit(s) formulations each time, 1-4 times per day
or
b) with 2-4 unit formulations, 2-4 times per day.

## Patentansprüche

1. Kombinationsarzneimittel, umfassend:
(i) mindestens ein Arzneimittel, ausgewählt aus Nitazoxanid oder Tizoxanid oder einem pharmazeutisch verträglichen Salz davon, mindestens ein Atovaquon oder ein pharmazeutisch verträgliches Salz davon und mindestens ein Ribavirin oder ein pharmazeutisch verträgliches Salz oder Ester davon; oder
(ii) mindestens ein Arzneimittel, ausgewählt aus Nitazoxanid oder Tizoxanid oder einem pharmazeutisch verträglichen Salz davon, und mindestens ein Atovaquon oder ein pharmazeutisch verträgliches Salz davon.

2. Kombinationsarzneimittel nach Anspruch 1, wobei das Kombinationsarzneimittel ferner mindestens ein antivirales Antikörper-Arzneimittel oder eine antivirale Antikörper-Arzneimittel-Kombination oder andere antivirale Arzneimittel umfasst.

3. Kombinationsarzneimittel nach Anspruch 1 oder 2 zur Verwendung in einem Verfahren zum Behandeln oder Vorbeugen einer Coronavirus-Infektion oder damit assoziierter Erkrankungen oder Zustände.

4. Kombinationsarzneimittel zur Verwendung nach Anspruch 3, wobei das Coronavirus ausgewählt ist aus der Gruppe bestehend aus SARS-CoV-2 oder seinen mutierten Stämmen, SARS, MERS, 229E, NL63, OC43 und HKU1 und wobei die Coronavirus-Infektion oder die damit assoziierten Erkrankungen oder Zustände mindestens eines der folgenden Symptome umfassen: Husten, verminderter Appetit, Asthma, Dyspnoe, Fieber, allgemeines Unwohlsein, Schwindel, Übelkeit, verminderter oder verlorener Geruchssinn und Pneumonie.

5. Kombinationsarzneimittel zur Verwendung nach Anspruch 4, wobei das Coronavirus SARS-CoV-2 oder dessen mutierte Stämme ist und die damit assoziierten Erkrankungen oder Zustände Pneumonie (COVID-19) ist, die durch Infektion mit SARS-CoV-2 oder dessen mutierten Stämmen verursacht wird.

6. Kombinationsarzneimittel zur Verwendung nach Anspruch 3, wobei das Verfahren Verabreichung von Nitazoxanid in einer oralen Formulierung, Verabreichung von Atovaquon in einer oralen Formulierung und Verabreichung von Ribavirin in einer oralen Formulierung, einer Inhalationsformulierung oder einer Injektionsformulierung umfasst.

7. Kombinationsarzneimittel zur Verwendung nach Anspruch 6, wobei das Verfahren Verabreichen von Nitazoxanid, Atovaquon und Ribavirin an ein behandlungsbedürftiges Subjekt umfasst, wobei Nitazoxanid einem behandlungsbedürftigen Subjekt in einer täglichen Gesamtdosis in dem Bereich von 100 mg bis 3000 mg verabreicht wird, wobei Atovaquon einem behandlungsbedürftigen Subjekt in einer täglichen Gesamtdosis in dem Bereich von 20 mg bis 3000 mg verabreicht wird, wobei Ribavirin einem behandlungsbedürftigen Subjekt in einer täglichen Gesamtdosis in dem Bereich von 20 mg bis 2500 mg verabreicht wird;
wobei Nitazoxanid, Atovaquon und Ribavirin einmal täglich (QD), zweimal täglich (BID), dreimal täglich (TID) oder viermal täglich (QID) verabreicht werden können.

8. Pharmazeutische Zusammensetzung, umfassend einen pharmazeutischen Wirkstoff und mindestens einen pharmazeutisch verträglichen Hilfsstoff, Träger oder Verdünnungsmittel; wobei der pharmazeutische Wirkstoff Folgendes umfasst:
(i):
a) mindestens ein Arzneimittel, ausgewählt aus Nitazoxanid oder Tizoxanid oder einem pharmazeutisch verträglichen Salz davon,
b) mindestens ein Atovaquon oder ein pharmazeutisch verträgliches Salz davon und c) mindestens ein Ribavirin oder ein pharmazeutisch verträgliches Salz oder Ester davon;
oder
(ii):
a) eine therapeutisch wirksame Menge Nitazoxanid oder eines pharmazeutisch verträglichen Salzes davon und
b) eine therapeutisch wirksame Menge an Atovaquon oder einem pharmazeutisch verträglichen Salz davon.

9. Pharmazeutische Zusammensetzung nach Anspruch 8 (i), wobei der pharmazeutische Wirkstoff Nitazoxanid, Atovaquon und Ribavirin umfasst und wobei das Gewichtsverhältnis von Nitazoxanid:Atovaquon:Ribavirin ausgewählt ist aus der Gruppe bestehend aus (0,1~10):(0,1~10):1, 1,5:1,5:1, 2:2:1, 2,5:2,5:1, 3:3:1, 10:10:3, 3,5:3,5:1, 4:4:1, 4,5:4,5:1, 5:5:1 und einem beliebigen Bereichswert zwischen beliebigen zwei der vorgenannten Verhältnisse.

10. Pharmazeutische Zusammensetzung nach Anspruch 9, wobei die pharmazeutische Zusammensetzung umfasst
(i) 50 mg bis 1200 mg Nitazoxanid pro Formulierungseinheit, 50 mg bis 1200 mg Atovaquon pro Formulierungseinheit und 1 mg bis 500 mg Ribavirin pro Formulierungseinheit;
(ii) 60 mg bis 600 mg Nitazoxanid pro Formulierungseinheit, 60 mg bis 600 mg Atovaquon pro Formulierungseinheit und 10 mg bis 200 mg Ribavirin pro Formulierungseinheit; oder
(iii) 111 mg Nitazoxanid pro Formulierungseinheit, 111 mg Atovaquon pro Formulierungseinheit und 33 mg Ribavirin pro Formulierungseinheit.

11. Pharmazeutische Zusammensetzung nach Anspruch 8 (ii), wobei der pharmazeutische Wirkstoff aus Nitazoxanid und Atovaquon in einem feststehenden Verhältnis besteht.

12. Pharmazeutische Zusammensetzung nach Anspruch 11, wobei das feststehende Verhältnis von Nitazoxanid und Atovaquon das Gewichtsverhältnis von Nitazoxanid zu Atovaquon ist, wobei das Gewichtsverhältnis von Nitazoxanid zu Atovaquon ausgewählt ist aus der Gruppe bestehend aus 1:0,1 bis 1:10, 1:0,1, 1:0,2, 1:0,5, 1:0,8, 1:1, 1:1,2, 1:1,5, 1:2, 1:5, 1:10 und einem beliebigen Bereichswert zwischen zwei beliebigen der vorgenannten Verhältnisse.

13. Pharmazeutische Zusammensetzung nach Anspruch 8, wobei die pharmazeutische Zusammensetzung als Tablette, Kapsel oder Suspension formuliert ist.

14. Pharmazeutische Zusammensetzung nach Anspruch 11, wobei die pharmazeutische Zusammensetzung umfasst:
(i) 50 mg bis 1200 mg Nitazoxanid pro Formulierungseinheit und 50 mg bis 1200 mg Atovaquon pro Formulierungseinheit;
(ii) 60 mg bis 500 mg Nitazoxanid pro Formulierungseinheit und 100 mg bis 500 mg Atovaquon pro Formulierungseinheit; oder
(iii) 165 mg Nitazoxanid pro Formulierungseinheit und 165 mg Atovaquon pro Formulierungseinheit.

15. Pharmazeutische Zusammensetzung nach einem der Ansprüche 8 bis 14 zur Verwendung in einem Therapieverfahren, wobei das Verfahren Verabreichen der pharmazeutischen Zusammensetzung an ein behandlungsbedürftiges Subjekt umfasst:
a) mit jeweils 1-5 Einheitsformulierungen, 1-4 Mal pro Tag oder
b) mit Formulierungen von 2-4 Einheiten, 2-4 Mal pro Tag.

## Revendications

1. Médicament combinatoire comprenant :
(i) au moins un médicament choisi parmi le nitazoxanide ou le tizoxanide ou un sel pharmaceutiquement acceptable de celui-ci, au moins une atovaquone ou un sel pharmaceutiquement acceptable de celle-ci, et au moins une ribavirine ou un sel ou ester pharmaceutiquement acceptable de celle-ci ; ou
(ii) au moins un médicament choisi parmi le nitazoxanide ou le tizoxanide ou un sel pharmaceutiquement acceptable de celui-ci, et au moins une atovaquone ou un sel pharmaceutiquement acceptable de celui-ci.

2. Médicament combinatoire selon la revendication 1, dans lequel le médicament combinatoire comprend en outre au moins un médicament à base d'anticorps antiviraux ou une combinaison de médicaments à base d'anticorps antiviraux, ou d'autres médicaments antiviraux.

3. Médicament combinatoire selon la revendication 1 ou 2 pour une utilisation dans une méthode de traitement ou de prévention de l'infection par le coronavirus ou des maladies ou affections qui y sont associées.

4. Médicament combinatoire pour l'utilisation selon la revendication 3, dans lequel le coronavirus est choisi parmi le groupe constitué du SARS-CoV-2 ou de ses souches mutantes, SARS, MERS, 229E, NL63, OC43 et HKU1, et dans lequel l'infection par le coronavirus ou les maladies ou affections qui y sont associées comprennent au moins un des symptômes suivants : toux, diminution de l'appétit, asthme, dyspnée, fièvre, malaise général, étourdissements, nausées, diminution ou perte de l'odorat et pneumonie.

5. Médicament combinatoire pour l'utilisation selon la revendication 4, dans lequel le coronavirus est le SARS-CoV-2 ou ses souches mutantes, et les maladies ou affections associées sont la pneumonie (COVID-19) causée par l'infection par le SARS-CoV-2 ou ses souches mutantes.

6. Médicament combinatoire pour l'utilisation selon la revendication 3, dans lequel la méthode comprend l'administration de nitazoxanide dans une formulation orale, l'administration d'atovaquone dans une formulation orale et l'administration de ribavirine dans une formulation orale, une formulation pour inhalation ou une formulation injectable.

7. Médicament combinatoire pour l'utilisation de la revendication 6, dans lequel la méthode comprend l'administration de nitazoxanide, d'atovaquone et de ribavirine à un sujet qui en a besoin, dans lequel le nitazoxanide est administré à un sujet qui en a besoin à une dose quotidienne totale allant de 100 mg à 3 000 mg, dans lequel l'atovaquone est administrée à un sujet qui en a besoin à une dose quotidienne totale allant de 20 mg à 3 000 mg, dans lequel la ribavirine est administrée à un sujet qui en a besoin à une dose quotidienne totale allant de 20 mg à 2 500 mg ;
dans lequel le nitazoxanide, l'atovaquone et la ribavirine peuvent être administrés respectivement une fois par jour (QD), deux fois par jour (BID), trois fois par jour (TID) ou quatre fois par jour (QID).

8. Composition pharmaceutique comprenant un ingrédient pharmaceutique actif et au moins un excipient, support ou diluant pharmaceutiquement acceptable ; dans laquelle l'ingrédient pharmaceutique actif comprend :
(i) :
a) au moins un médicament choisi parmi le nitazoxanide ou le tizoxanide ou un sel pharmaceutiquement acceptable de celui-ci,
b) au moins une atovaquone ou un sel pharmaceutiquement acceptable de celle-ci, et c) au moins une ribavirine ou un sel ou ester pharmaceutiquement acceptable de celle-ci ;
ou
(ii) :
a) une quantité thérapeutiquement efficace de nitazoxanide ou d'un sel pharmaceutiquement acceptable de celui-ci et
b) une quantité thérapeutiquement efficace d'atovaquone ou d'un sel pharmaceutiquement acceptable de celle-ci.

9. Composition pharmaceutique selon la revendication 8 (i), dans laquelle l'ingrédient pharmaceutique actif comprend du nitazoxanide, de l'atovaquone et de la ribavirine, et dans laquelle le rapport pondéral nitazoxanide:atovaquone:ribavirine est choisi parmi le groupe constitué de (0,1~10):(0,1~10):1, 1,5:1,5:1, 2:2:1, 2,5:2,5:1, 3:3:1, 10:10:3, 3,5:3,5:1, 4:4:1, 4,5:4,5:1, 5:5:1, et toute valeur comprise entre deux rapports quelconques ci-dessus.

10. Composition pharmaceutique selon la revendication 9, dans laquelle la composition pharmaceutique comprend
(i) 50 mg à 1 200 mg de nitazoxanide par unité de formulation, 50 mg à 1 200 mg d'atovaquone par unité de formulation et 1 mg à 500 mg de ribavirine par unité de formulation ;
(ii) 60 mg à 600 mg de nitazoxanide par unité de formulation, 60 mg à 600 mg d'atovaquone par unité de formulation et 10 mg à 200 mg de ribavirine par unité de formulation ; ou
(iii) 111 mg de nitazoxanide par unité de formulation, 111 mg d'atovaquone par unité de formulation et 33 mg de ribavirine par unité de formulation.

11. Composition pharmaceutique de la revendication 8 (ii), dans laquelle l'ingrédient pharmaceutique actif est constitué de nitazoxanide et d'atovaquone dans un rapport fixe.

12. Composition pharmaceutique selon la revendication 11, dans laquelle le rapport fixe de nitazoxanide et d'atovaquone est le rapport pondéral nitazoxanide:atovaquone, dans laquelle le rapport pondéral est choisi parmi le groupe constitué de 1:0,1 à 1:10, 1:0,1, 1:0,2, 1:0,5, 1:0,8, 1:1, 1:1,2, 1:1,5, 1:2, 1:5, 1:10, et toute valeur comprise entre deux rapports quelconques ci-dessus.

13. Composition pharmaceutique selon la revendication 8, dans laquelle la composition pharmaceutique est formulée sous forme de comprimé, de gélule ou de suspension.

14. Composition pharmaceutique selon la revendication 11, dans laquelle la composition pharmaceutique comprend :
(i) 50 mg à 1 200 mg de nitazoxanide par unité de formulation, et 50 mg à 1 200 mg d'atovaquone par unité de formulation ;
(i) 60 mg à 500 mg de nitazoxanide par unité de formulation, et 100 mg à 500 mg d'atovaquone par unité de formulation ; ou (iii) 165 mg de nitazoxanide par unité de formulation et 165 mg d'atovaquone par unité de formulation.

15. Composition pharmaceutique selon l'une quelconque des revendications 8 à 14 pour une utilisation dans une méthode thérapeutique, dans laquelle la méthode comprend l'administration de la composition pharmaceutique à un sujet qui en a besoin :
a) avec 1 à 5 unités de formulation à chaque fois, 1 à 4 fois par jour ou
b) avec 2 à 4 unités de formulation, 2 à 4 fois par jour.
